# EUROPEAN PATENT APPLICATION

(11) **EP 1 291 679 A1**
(43) Date of publication of application: **12.03.2003**
(21) Application number: 01934387.0
(22) Date of filing: 29.05.2001
(51) Int. Cl.: G02B 1/04

(54) **OPTICAL MATERIAL AND OPTICAL PART EACH CONTAINING AROMATIC SULFIDE COMPOUND AND AROMATIC SULFIDE COMPOUND**

(30) Priority: 31.05.2000 JP 2000161348; 07.12.2000 JP 2000372499
(71) Applicant: Mitsui Chemicals, Inc., Tokyo 100-6070 (JP)
(72) Inventor: FUJIYAMA, Takahiro, Sodegaura-shi, Chiba 299-0265 (JP); HAMA, Hideo, Sodegaura-shi, Chiba 299-0265 (JP); OTSUJI, Atsuo, Sodegaura-shi, Chiba 299-0265 (JP); TAKUMA, Keisuke, Chiyoda-ku, Tokyo 100-6070 (JP)
(74) Representative: Stuart, Ian Alexander
(86) International application number: JP0104491
(87) International publication number: WO01092925

(57) **Abstract**

Optical materials with improved heat resistance, especially dopant-type GI POFs with improved heat resistance are provided. These optical materials each comprises at least one aromatic sulfide compound represented by the following formula (1): 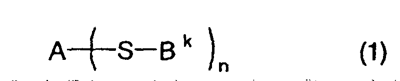 wherein
n stands for an integer of from 2 to 12,
k stands for an integer of from 1 to n,
A represents a substituted or unsubstituted, n-valent carbocyclic aromatic ring or heterocyclic aromatic ring, and
B¹ to Bⁿ each independently represent a substituted or unsubstituted, carbocyclic aromatic group or heterocyclic aromatic group.

## Description

### TECHNICAL FIELD

This invention relates to optical materials and optical parts, which make use of aromatic sulfide compounds, and also to the aromatic sulfide compounds. Especially, the present invention is concerned with polymer optical fibers.

### BACKGROUND ART

As optical material, glass has been used traditionally. In recent years, however, transparent polymer materials have begun to find wide-spread utility. In particular, they are used in fields such as optical lens, optical disks, optical fibers, rod lenses, optical waveguides, optical switches, and optical pickup lenses. Optical polymer materials have been developed in pursuit of higher functions such as higher transparency, higher refractive index, lower dispersion, lower birefringence and higher heat resistance. Among these, polymer optical fibers (POFs) are attracting increasing importance in the next generation communication network conception such as LAN (local area network) and ISDN (integrated service ' digital network).

In POF, a core part and a cladding part are both formed of a polymer. Compared with silica glass optical fibers, POFs permit easier processing and handling and require less costly materials. POFs are, therefore, widely used as optical transmission lines for such short distances that transmission losses cause no practical problem.

Step index (SI) POFs in each of which the refractive index distribution varies stepwise have already been put into practical use for transmission over a short distance of 50 m or so, but are not suited for optical communications due to small transmission capacity. On the other hand, grated index (GI) POFs in each of which the refractive index distribution varies in a radial direction are greater in transmission capacity than SI POFs and are suited for optical communications, and the smoother the refractive index distribution, the greater the transmission capacity of a fiber.

As manufacturing processes of GI POFs, there are two types of processes. One of these two types of processes is of the dopant type such as that disclosed, for example, in WO93/08488. According to the dopant-type process, a refractive index distribution is obtained by adding, to a matrix polymer, a low molecular weight compound having no reactivity to the polymer and causing the low molecular weight compound to diffuse such that a concentration gradient is formed. The other is of the copolymerization type disclosed, for example, in JP 5-173025 A or JP 5-173026 A. According to this copolymerization-type process, a refractive index distribution is obtained by forming a concentration gradient while making use of a difference in reactivity between two monomers upon copolymerization of these monomers.

However, the copolymerization-type process can hardly avoid occurrence of a microscopic non-uniform structure due to differences in the copolymerized composition and tends to develop a problem in transparency by the microscopic non-uniform structure. Accordingly, the transmission distance available under the current situation is limited to about 50 m, so that transmission distances required for domestic LAN and the like cannot be fully met. The dopant-type process, on the other hand, can provide extremely high transparency to wavelengths as the size of the dopant is on the order of several Å, but involves a problem in heat resistance. When used under an atmosphere the temperature of which is higher than a certain temperature, the distribution of the dopant tends to vary, leading to a problem that the refractive index distribution tends to vary and the heat stability of the refractive index distribution is inferior.

This problem can be attributed to a reduction in the glass transition temperature of the core material by the plasticization effect of the dopant. The glass transition temperature of PMMA which has been used in conventional POFs is around 105°C. When a dopant is added, the glass transition temperature drops to around room temperature. For example, benzyl benzoate, benzyl-n-butyl phthalate, benzyl salicylate, bromobenzene, benzyl phenyl ether, diphenyl phthalate, diphenylmethane, diphenyl ether, diphenyl, diphenyl sulfide, phenyl benzoate, triphenyl phosphate, tricresyl phosphate and the like are known as dopants for GI POFs. Among these, diphenyl sulfide is disclosed to bring about both plasticizing effect and higher refractive index in JP-A 11-142657. Nonetheless, even use of this dopant is still unable to fully satisfy the heat resistance.

### DISCLOSURE OF THE INVENTION

The present invention has been completed with the foregoing circumstances in view and has as an object the provision of an aromatic sulfide compound useful for an optical material. More specifically, an object of the present invention is to provide a dopant-type GI POF improved in heat resistance.

The present inventors have devoted themselves to ' the solution of the above-described problems, and have found that use of an aromatic sulfide of a particular structure as a dopant for an optical material makes it possible to inhibit a reduction in the glass transition temperature of a core material, to improve the heat resistance and hence to permit using under an atmosphere of a temperature higher than a certain temperature, leading to the completion of the present invention.

The present invention, therefore, provides:
[1] An optical material comprising at least one aromatic sulfide compound represented by the following formula (1): 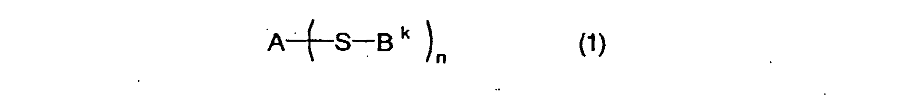 wherein
   n stands for an integer of from 2 to 12,
   k stands for an integer of from 1 to n,
   A represents a substituted or unsubstituted, n-valent carbocyclic aromatic ring or heterocyclic aromatic ring, and
   B¹ to Bⁿ each independently represent a substituted or unsubstituted, carbocyclic aromatic group or heterocyclic aromatic group.
[2] An optical material as described above under [1], wherein in formula (1), n stands for an integer of from 2 to 4, and A is a substituted or unsubstituted, heterocyclic aromatic ring.
[3] An optical material as described above under [2], wherein in formula (1), B¹ to Bⁿ each independently are a substituted or unsubstituted phenyl group, a substituted or unsubstituted pyrimidyl group, a substituted or unsubstituted naphthyl group, a substituted or unsubstituted thienyl group, a substituted or unsubstituted benzothiazolyl group, a substituted or unsubstituted benzoxazolyl group, a substituted or unsubstituted thiadiazolyl group, or a substituted or unsubstituted thiazolyl group.
[4] An optical material as described above under [2], wherein in formula (1), A is a divalent heterocyclic aromatic ring selected from a substituted or unsubstituted thiophene ring, a substituted or unsubstituted thiophene-1,1-dioxide ring, a substituted or unsubstituted thiophenethiadiazole ring, a substituted or unsubstituted thieno[3,2-b]thiophene ring, a substituted or unsubstituted triazine ring, or a substituted or unsubstituted pyrimidine ring.
[5] An optical material as described above under [4], wherein in formula (1), B¹ to Bⁿ each independently are a substituted or unsubstituted phenyl group, a substituted or unsubstituted pyrimidyl group, a substituted or unsubstituted naphthyl group, a substituted or unsubstituted thienyl group, a substituted or unsubstituted benzothiazolyl group, a substituted or unsubstituted benzoxazolyl group, a substituted or unsubstituted thiadiazolyl group, or a substituted or unsubstituted thiazolyl group.
[6] An optical material as described above under [2], wherein in formula (1), A is a trivalent heterocyclic aromatic ring selected from a substituted or unsubstituted thiophene ring, a substituted or unsubstituted triazine ring, or a substituted or unsubstituted pyrimidine ring.
[7] An optical material as described above under [6], wherein in formula (1), B¹ to Bⁿ each independently are a substituted or unsubstituted phenyl group, a substituted or unsubstituted pyrimidyl group, a substituted or unsubstituted naphthyl group, a substituted or unsubstituted thienyl group, a substituted or unsubstituted benzothiazolyl group, a substituted or unsubstituted benzoxazolyl group, a substituted or unsubstituted thiadiazolyl group, or a substituted or unsubstituted thiazolyl group.
[8] An optical material as described above under [2], wherein in formula (1), A is a tetravalent heterocyclic aromatic ring selected from a substituted or unsubstituted thiophene ring or a substituted or unsubstituted thieno[3,2-b]thiophene ring.
[9] An optical material as described above under [8], wherein in formula (1), B¹ to Bⁿ each independently are a substituted or unsubstituted phenyl group, a substituted or unsubstituted pyrimidyl group, a substituted or unsubstituted naphthyl group, a substituted or unsubstituted thienyl group, a substituted or unsubstituted benzothiazolyl group, a substituted or unsubstituted benzoxazolyl group, a substituted or unsubstituted thiadiazolyl group, or a substituted or unsubstituted thiazolyl group.
[10] An optical material as described above under [1], wherein in formula (1), n stands for an integer of from 2 to 6, and A is a substituted or unsubstituted, carbocyclic aromatic ring.
[11] An optical material as described above under [10], wherein in formula (1), B¹ to Bⁿ each independently are a substituted or unsubstituted phenyl group, a substituted or unsubstituted pyrimidyl group, a substituted or unsubstituted naphthyl group, a substituted or unsubstituted thienyl group, a substituted or unsubstituted benzothiazolyl group, a substituted or unsubstituted benzoxazolyl group, a substituted or unsubstituted thiadiazolyl group, or a substituted or unsubstituted thiazolyl group.
[12] An optical material as described above under [10], wherein in formula (1), A is a divalent carbocyclic aromatic ring selected from a substituted or unsubstituted benzene ring, a substituted or unsubstituted naphthalene ring, a substituted or unsubstituted fluorene ring, or a substituted or unsubstituted biphenyl group.
[13] An optical material as described above under [12], wherein in formula (1), B¹ to Bⁿ each independently are a substituted or unsubstituted phenyl group, a substituted or unsubstituted pyrimidyl group, a substituted or unsubstituted naphthyl group, a substituted or unsubstituted thienyl group, a substituted or unsubstituted benzothiazolyl group, a substituted or unsubstituted benzoxazolyl group, a substituted or unsubstituted thiadiazolyl group, or a substituted or unsubstituted thiazolyl group.
[14] An optical material as described above under [10], wherein in formula (1), A is a trivalent carbocyclic aromatic ring selected from a substituted or unsubstituted benzene ring or a substituted or unsubstituted fluorene ring.
[15] An optical material as described above under [14], wherein in formula (1), B¹ to Bⁿ each independently are a substituted or unsubstituted phenyl group, a substituted or unsubstituted pyrimidyl group, a substituted or unsubstituted naphthyl group, a substituted or unsubstituted thienyl group, a substituted or unsubstituted benzothiazolyl group, a substituted or unsubstituted benzoxazolyl group, a substituted or unsubstituted thiadiazolyl group, or a substituted or unsubstituted thiazolyl group.
[16] An optical material as described above under [10], wherein in formula (1), A is a tetravalent carbocyclic aromatic ring selected from a substituted or unsubstituted benzene ring or a substituted or unsubstituted biphenyl group.
[17] An optical material as described above under [16], wherein in formula (1), B¹ to Bⁿ each independently are a substituted or unsubstituted phenyl group, a substituted or unsubstituted pyrimidyl group, a substituted or unsubstituted naphthyl group, a substituted or unsubstituted thienyl group, a substituted or unsubstituted benzothiazolyl group, a substituted or unsubstituted benzoxazolyl group, a substituted or unsubstituted thiadiazolyl group, or a substituted or unsubstituted thiazolyl group.
[18] An optical material as described above under [1] to [17], which is a polymer optical fiber material.
[19] An optical material as described above under [1] to [17], which is formed in a polymer optical fiber.
[20] An optical material as described above under [1] to [17], which is formed in a GI polymer optical fiber.
[21] An aromatic sulfide compound represented by the following formula (1a): 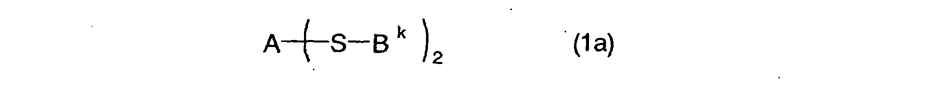 wherein
   k stands for an integer of from 1 to 2,
   A represents a divalent carbocyclic aromatic ring or heterocyclic aromatic ring selected from a substituted or unsubstituted benzene ring, a substituted or unsubstituted naphthalene ring, a substituted or unsubstituted fluorene ring, a substituted or unsubstituted biphenyl ring, a substituted or unsubstituted thiophene ring, a substituted or unsubstituted thiophene-1,1-dioxide ring, a substituted or unsubstituted thiophenethiadiazole ring, a substituted or unsubstituted thieno[3,2-b]thiophene ring, a substituted or unsubstituted triazine ring, or a substituted or unsubstituted pyrimidine ring, and
   B¹ and B² each independently represent a carbocyclic aromatic group or heterocyclic aromatic group selected from a substituted or unsubstituted phenyl group, a substituted or unsubstituted pyrimidyl group, a substituted or unsubstituted naphthyl group, a substituted or unsubstituted thienyl group, a substituted or unsubstituted benzothiazolyl group, a substituted or unsubstituted benzoxazolyl group, a substituted or unsubstituted thiadiazolyl group, or a substituted or unsubstituted thiazolyl group.
[22] An aromatic sulfide compound represented by the following formula (1b): 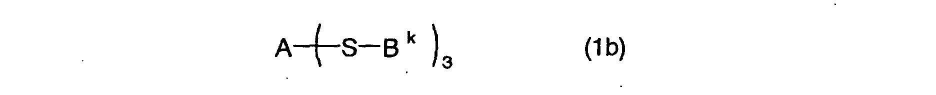 wherein
   k stands for an integer of from 1 to 3,
   A represents a trivalent carbocyclic aromatic ring or heterocyclic aromatic ring selected from a substituted or unsubstituted benzene ring, a substituted or unsubstituted fluorene ring, a substituted or unsubstituted thiophene ring, a substituted or unsubstituted triazine ring, or a substituted or unsubstituted pyrimidine ring, and
   B¹, B² and B³ each independently represent a carbocyclic aromatic group or heterocyclic aromatic group selected from a substituted or unsubstituted phenyl group, a substituted or unsubstituted pyrimidyl group, a substituted or unsubstituted naphthyl group, a substituted or unsubstituted thienyl group, a substituted or unsubstituted benzothiazolyl group, a substituted or unsubstituted benzoxazolyl group, a substituted or unsubstituted thiadiazolyl group, or a substituted or unsubstituted thiazolyl group.
[23] An aromatic sulfide compound represented by the following formula (1c): 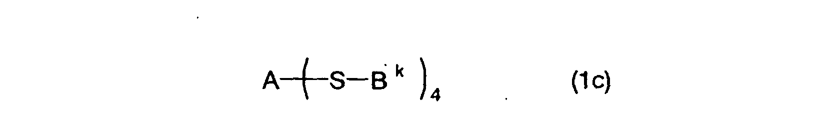 wherein
   k stands for an integer of from 1 to 4,
   A represents a carbocyclic aromatic ring or heterocyclic aromatic ring selected from a substituted or unsubstituted benzene ring, a substituted or unsubstituted biphenyl ring, a substituted or unsubstituted thiophene ring, a substituted or unsubstituted thieno[3,2-b]thiophene ring, and
   B¹, B², B³ and B⁴ each independently represent a carbocyclic aromatic group or heterocyclic aromatic group selected from a substituted or unsubstituted phenyl group, a substituted or unsubstituted pyrimidyl group, a substituted or unsubstituted naphthyl group, a substituted or unsubstituted thienyl group, a substituted or unsubstituted benzothiazolyl group, a substituted or unsubstituted benzoxazolyl group, a substituted or unsubstituted thiadiazolyl group, or a substituted or unsubstituted thiazolyl group.

### BRIEF DESCRIPTION OF THE DRAWINGS

FIG. 1 is a graph showing variations in the refractive indexes of spin-coated films depending upon variations in the concentrations of dopants as measured in Example 8 and Comparative Example 1; and
FIG. 2 is a graph showing relationships between ' glass transition temperature and refractive index as measured in Example 15 and Comparative Example 2.

### BEST MODES FOR CARRYING OUT THE INVENTION

The present invention will hereinafter be described in detail.

The optical material according to the present invention is characterized by comprising:
(a) a transparent polymer, and
(b) an aromatic sulfide compound.

The optical material according to the present invention is characterized by comprising at least one aromatic sulfide compound represented by the following formula (1): 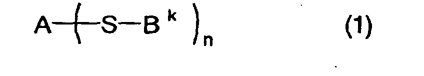 wherein
n stands for an integer of from 2 to 12,
k stands for an integer of from 1 to n,
A represents a substituted or unsubstituted, n-valent carbocyclic aromatic ring or heterocyclic aromatic ring, and
B¹ to Bⁿ each independently represent a substituted or unsubstituted, carbocyclic aromatic group or heterocyclic aromatic group.

A description will firstly be made about A in formula (1), which forms a central skeleton.

In the heterocyclic aromatic ring, the aromatic ring is composed of atoms of two or more elements. Illustrative of the atoms of two or more elements are carbon atom, oxygen atom, phosphorus atom, sulfur atom, and nitrogen atom. The aromatic ring may preferably be composed of atoms of 2 to 5 types of elements, with atoms of 2 to 4 types of elements being more preferred. It is to be noted that atoms other than carbon atom will be referred to as "hetero atoms".

The heterocyclic aromatic ring may be composed of either a 5-membered ring or a 6-membered ring. The heterocyclic aromatic ring may preferably be composed of a single ring or 2 to 4 aromatic rings fused together, with a single ring or 2 to 3 aromatic rings fused together being more preferred.

The number of carbons contained in the heterocyclic aromatic ring may preferably be 4 to 14, with 4 to 11 being more preferred.

More preferred specific examples of the heterocyclic aromatic ring will be described.

Firstly, 5-membered rings each of which are represented by the following formula (2) and contain one hetero atom can be mentioned. Illustrative of formula (2) are furan ring (Z=O), thiophene ring (Z=S) and pyrrole ring (Z=NH). Among these, preferred are thiophene ring and furan ring, and more preferred is thiophene ring.

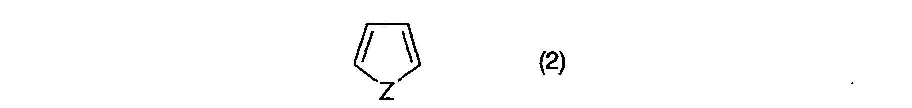

Further, the following formula (3) with a benzene ring fused with formula (2) can be mentioned. Illustrative of formula (3) are indole ring (Z=NH), benzofuran ring (Z=O) and benzothiophene ring (Z=S).

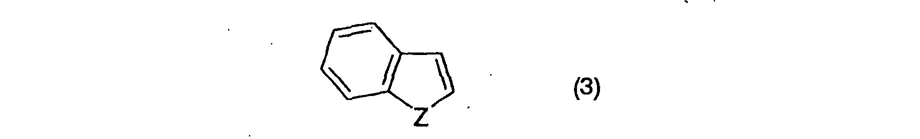

In addition, the following formulas (4a) to (4f) with aromatic rings fused with a thiophene ring of formula (2) in which Z=S can also be mentioned. Among these, preferred are isothianaphthene ring, thienothiadiazole ring and thieno[3,2-b]thiophene ring, and more preferred are thienothiadiazole ring and thieno[3,2-b]thiophene ring.

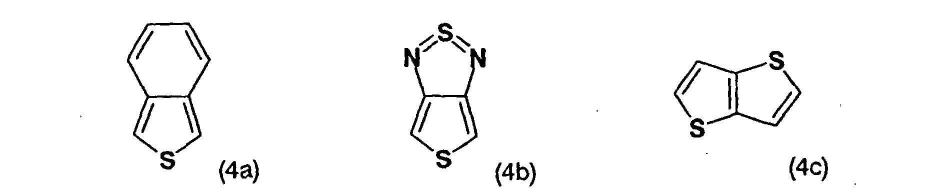

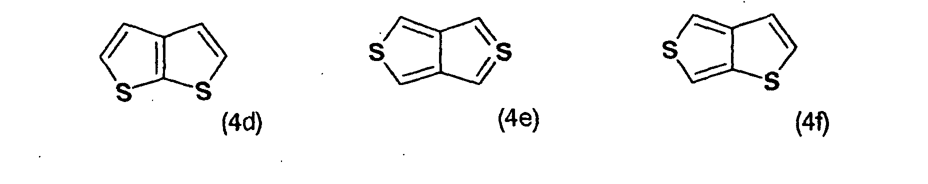

Next, 5-membered rings each of which are represented by the following formula (5a) or (5b) and contain two hetero atoms can be mentioned. Illustrative of formula (5a) are oxazole ring (Z¹=O), thiazole ring (Z¹=S) and imidazole ring (Z¹=NH). Illustrative of formula (5b) are isoxazole ring (Z²=O), isothiazole ring (Z²=S) and pyrazole ring (Z²=NH}. Among these, oxazole ring and thiazole ring are preferred, with thiazole ring being more preferred.

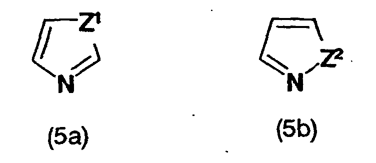

Further, the following general formula (6a) or (6b) with a benzene ring fused with the 5-membered ring of formula (5a) or (5b) can be mentioned. Illustrative of formula (6a) are benzoxazole ring (Z¹=O), benzothiazole ring (Z¹=S) and benzimidazole ring (Z¹=NH). Illustrative of formula (6b) are benzisoxazole ring (Z²=O), benzisothiazole ring (Z²=S) and benzopyrazole ring (Z²=NH).

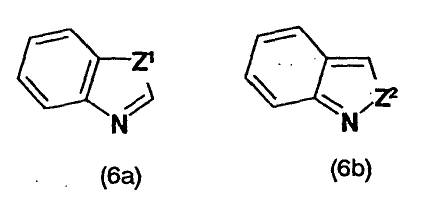

Examples of 5-membered rings each of which contains 3 or more hetero atoms include n-triazole ring, s-triazole ring, 1,2,4-oxadiazole ring, 1,3,5-oxadiazole ring, 1,2,5-oxadiazole ring, 1,2,4-thiadiazole ring, 1,3,5-thiadiazole ring, 1,2,5-thiadiazole ring and tetrazole ring. Among these, preferred are 1,3,5-oxadiazole ring and 1,3,5-thiadiazole ring, with 1,3,5-thiadiazole ring being more preferred.

As a 6-membered ring which contains one hetero atom, pyridine ring can be mentioned. In addition, quinoline ring and isoquinoline ring each of which contains a pyridine ring and a benzene ring fused therewith can also be mentioned.

Next, examples of 6-membered rings each of which contains two hetero atoms include pyridazine ring, pyrimidine ring and pyrazine ring. Benzo[d]pyridazine ring, benzo[c]pyridazine ring, quinazoline ring and quinoxaline can also be mentioned, each of which is composed of such a 6-membered ring and a benzene ring fused therewith.

Further, as a 6-membered ring containing three hetero atoms, triazine ring can be mentioned.

In formula (1), A can be polycyclic. Described specifically, it can be a polycyclic ring formed of plural rings one-dimensionally connected together by single bonds, respectively, as indicated by the below-described formula (7).

Here, Z represents an oxygen atom or a sulfur atom. On the other hand, m stands for an integer of from 0 to 2. Of these, Z is preferably a sulfur atom. The preferred value of m is 0 or 1, with 0 being a more preferred value.

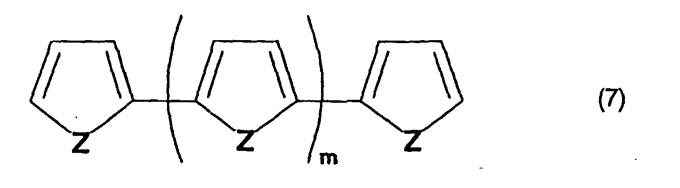

The carbocyclic aromatic ring represented by A in formula (1) is a cyclic compound in which the atoms constituting an aromatic ring are all carbon atoms. The carbocyclic aromatic ring is formed of a 5-membered ring or a 6-mebered ring. The carbocyclic aromatic ring may preferably be composed of a single ring or 2 to 5 aromatic rings fused together, with a single ring or 2 to. 4 aromatic rings fused together being more preferred.

The number of carbon atoms contained in the carbocyclic aromatic ring may preferably range from 6 to 22, with a range of from 6 to 18 being more preferred.

As specific examples of such carbocyclic aromatic rings, fused polycyclic aromatic rings can be mentioned. Illustrative are pentalene ring, phenalene ring, triphenylene ring, perylene ring, indene ring, azulene ring, phenanthrene ring, pyrene ring, and picene ring. Among these, acene-type aromatic rings are preferred. Specific examples include benzene ring, naphthalene ring, anthracene ring, napthacene ring, and pentacene ring. Among these, more preferred are benzene ring, naphthalene ring and anthracene ring, with benzene ring and naphthalene ring being still more preferred.

In formula (1), A can be polycyclic. Described specifically, it can be a polycyclic ring formed of plural rings one-dimensionally connected together by single bonds, respectively, as indicated by the below-described formula (8). Here, m is an integer of from 0 to 2. The preferred value of m is 0 or 1, with 0 being a more preferred value.

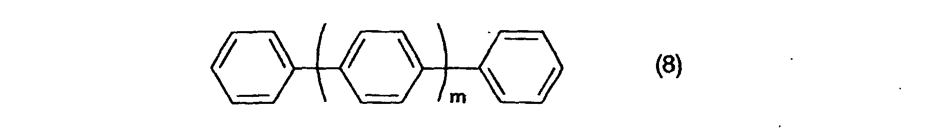

In formula (1), A may be of such a structure as illustrated by the below-described formula (9). Specific examples include carbazole ring (Z³=NH), dibenzofuran ring (Z³=O), dibenzothiphene ring (Z³=S), fluorene ring (Z³=CH₂), fluorenone ring (Z³=CO), and dibenzothiophensulfone ring (Z³=SO₂). Among these, preferred are dibenzothiophene ring, fluorene ring, fluorenone ring and dibenzothiophensulfone ring, with dibenzothiophene ring, fluorene and fluorenone ring being more preferred.

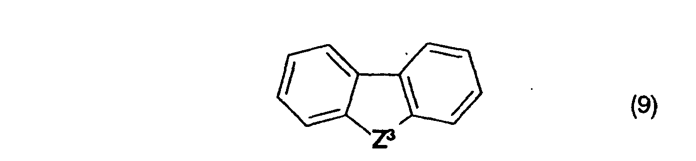

Preferred structures as the carbocyclic aromatic ring or heterocyclic aromatic ring represented by A are the following structural formulas:

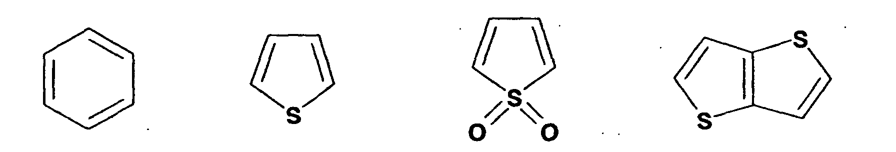

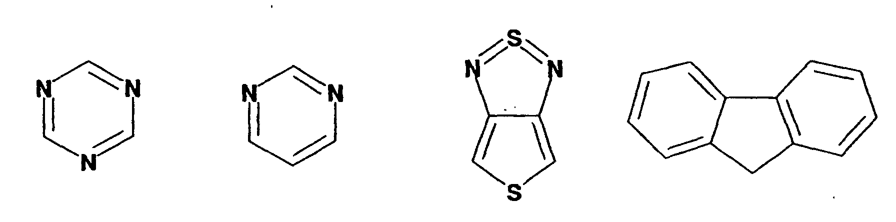

The heterocyclic aromatic ring or carbocyclic aromatic ring represented by A in formula (1) may contain one or more substituents. Illustrative of such substituents are alkyl groups, alkoxy groups, and halogen atoms.

As alkyl groups, alkyl groups having 1 to 4 carbon atoms are preferred. Specific preferred examples include linear alkyl groups such as methyl, ethyl, n-propyl and n-butyl, and branched alkyl groups such as isopropyl, s-butyl and t-butyl.

As alkoxy groups, alkoxy groups having 1 to 3 carbon atoms are preferred. Specific preferred examples include methoxy, ethoxy, propoxy, and isopropoxy.

Illustrative of halogen atoms are fluorine atoms, chlorine atoms, bromine atoms and iodine atoms, with fluorine atoms and chlorine atoms being preferred.

These substituents are chosen in view of the melting point, the compatibility with the associated polymer, and the like. If the compatibility with the associated polymer is poor, introduction of t-butyl group(s) or the like is effective.

A description will next be made about B¹ to Bⁿ in formula (1). B¹ to Bⁿ each independently represent a substituted or unsubstituted, carbocyclic or heterocyclic aromatic group.

In the heterocyclic aromatic group, the aromatic ring is composed of atoms of two or more elements. Illustrative of the atoms of two or more elements are carbon atom, oxygen atom, phosphorus atom, sulfur atom, and nitrogen atom. The aromatic ring may preferably be composed of atoms of 2 to 5 types of elements, with atoms of 2 to 4 types of elements being more preferred. Incidentally, atoms other than carbon atom will be referred to as "hetero atoms".

The heterocyclic aromatic group may be composed of either a 5-membered ring or a 6-membered ring. The heterocyclic aromatic group may preferably be composed of 1 to 4 aromatic rings fused together; with 1 to 3 aromatic rings fused together being more preferred.

The number of carbons contained in the heterocyclic aromatic group may preferably be 4 to 14, with 4 to 11 being more preferred.

More preferred specific examples of the heterocyclic aromatic group will be described. Firstly, 5-membered rings each of which is represented by the following formula (10) and contains one hetero atom can be mentioned. Illustrative of the formula (10) are furyl group (Z=O), thienyl group (Z=S) and pyrrolyl group (Z=NH). Among these, preferred are thienyl group and furyl group, and more preferred is thienyl group.

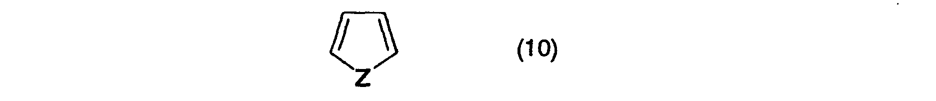

Further, the following formula (11) with a benzene ring fused with formula (10) can be mentioned. Illustrative of the formula (11) are indolyl group (Z=NH), benzofuryl group (Z=O) and benzothienyl group (Z=S). Among these, preferred are benzothienyl group and benzofuryl group, with benzothienyl group being more preferred.

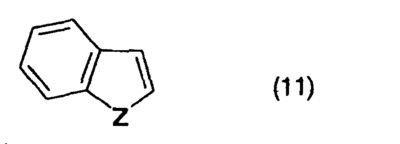

Next, 5-membered rings each of which is represented by the following formula (12a) or (12b) and contains two hetero atoms can be mentioned. Illustrative of formula' (12a) are oxazolyl group (Z¹=O), thiazolyl group (Z¹=S) and imidazolyl group (Z¹=NH). Illustrative of formula (12b) are isoxazolyl group (Z²=O), isothiazolyl group (Z²=S) and pyrazolyl group (Z²=NH). Among these, preferred are oxazolyl group and thiazolyl group, with thiazolyl group being more preferred.

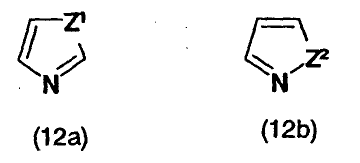

Further, the following general formula (13a) or (13b) with a benzene ring fused with the 5-membered ring of formula (12a) or (12b) can be mentioned. Illustrative of formula (13a) are benzoxazolyl group (Z¹=O), benzothiazolyl group (Z¹=S) and benzimidazolyl group (Z¹=NH). Illustrative of formula (13b) are benzisoxazolyl group (Z²=O), benzisothiazolyl group (Z²=S) and benzopyrazolyl (Z²=NH). Among these, preferred are benzothiazolyl and benzothiazoly, with benzothiazolyl being more preferred.

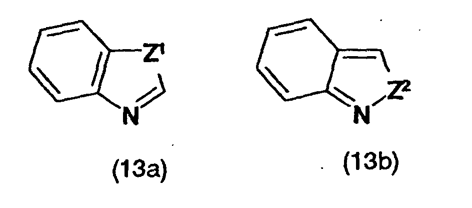

Examples of 5-membered rings each of which contains 3 or more hetero atoms include n-triazyl group, s-triazyl group, 1,2,4-oxadiazolyl group, 1,3,5-oxadiazolyl group, 1,2,5-oxadiazolyl group, 1,2,4-thiadiazolyl group, 1,3,5-thiadiazolyl group, 1,2,5-thiadiazolyl group and tetrazolyl group. Among these, preferred are 1,3,5-oxadiazolyl group and 1,3,5-thiadiazolyl group, with 1,3,5-thiadiazolyl group being more preferred.

As a 6-membered ring which contains one hetero atom, pyridyl group can be mentioned. In addition, quinolyl group and isoquinolyl group each of which contains a pyridyl group and a benzene ring fused therewith can also be mentioned. Preferred is pyridyl group.

Next, examples of 6-membered rings each of which contains two hetero atoms include pyridazyl group, pyrimidyl group and pyrazyl group. Benzo[d]pyridazyl group, benzo[c]pyridazyl group, quinazolyl group and quinoxalinyl group can also be mentioned, each of which is composed of such a 6-membered ring and a benzene ring fused therewith.

As a 6-membered ring containing three hetero atoms, triazyl group can be mentioned.

The carbocyclic aromatic group represented by B¹ to Bⁿ in formula (1) is a cyclic compound group in which the atoms constituting an aromatic ring are all carbon atoms. The carbocyclic aromatic group is formed of a 5-membered ring or a 6-mebered ring. The carbocyclic aromatic group may preferably be composed of a single ring or 2 to 5 ' aromatic rings fused together, with a single ring or 2 to 4 aromatic rings fused together being more preferred.

The number of carbon atoms contained in the carbocyclic aromatic group may preferably range from 6 to 22, with a range of from 6 to 18 being more preferred.

As specific examples of such carbocyclic aromatic groups, fused polycyclic aromatic groups can be mentioned. Illustrative are pentalenyl group, phenalenyl group, triphenylenyl group, perylenyl group, indenyl group, azulenyl group, phenanthrenyl group, pyrenyl group, and picenyl group. Among these, acene-type aromatic groups are preferred. Specific examples include phenyl group, naphthyl group, anthryl group, napthacenyl group, and pentacenyl group. Among these, preferred are phenyl group, naphthyl group-and anthracenyl group, with phenyl group and naphthyl group being more preferred.

Structures preferred as B¹ to Bⁿ in formula (1) are the following structural formulas.

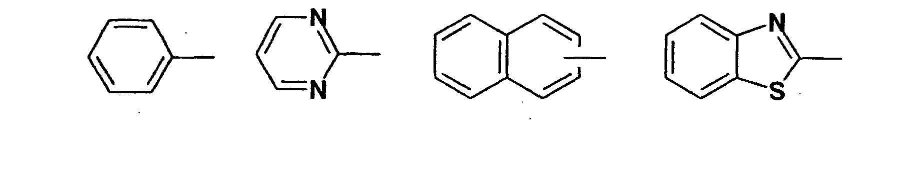

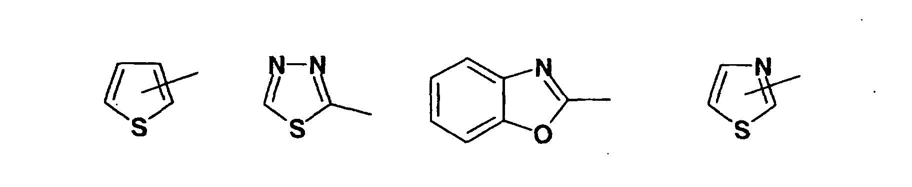

In formula (1), B¹ to Bⁿ may all be the same or may all be different.

The carbocyclic aromatic group or heterocyclic aromatic group represented by each of B¹ to Bⁿ may contain one or more substituents. Illustrative of such substituents are alkyl groups, alkoxy groups, and halogen atoms.

As alkyl groups, alkyl groups having 1 to 4 carbon atoms are preferred. Specific preferred examples include linear alkyl groups such as methyl, ethyl, n-propyl and n-butyl, and branched alkyl groups such as isopropyl, s-butyl and t-butyl. Particularly preferred are methyl, ethyl, n-butyl and t-butyl.
As alkoxy groups, alkoxy groups having 1 to 3 carbon atoms are preferred. Specific preferred examples include methoxy, ethoxy, propoxy, and isopropoxy, with methoxy being particularly preferred.

Illustrative of halogen atoms are fluorine atoms, chlorine atoms, bromine atoms and iodine atoms, with fluorine atoms and chlorine atoms being preferred. Particularly preferred is fluorine atom.

These substituents are chosen in view of the melting point, the compatibility with the associated polymer, and the like. If the compatibility with the associated polymer is poor, introduction of t-butyl group(s) or the like is effective. When a linear alkyl group is introduced to an asymmetrical position, for example, when a methyl group is introduced to an asymmetrical position of a phenyl group, the meta-position is more preferred than the para-position.

In formula (1), n stands for an integer of from 2 to 12. No particular limitation is imposed on n, because it is determined depending upon the molecular structure of A. When A is a heterocyclic aromatic ring, the preferred range of n is from 2 to 6, with a range of from 2 to 4 being more preferred. When A is a carbocyclic aromatic ring, on the other hand, the preferred range of n is from 2 to 10, with a range of from 2 to 6 being more preferred.

In formula (1), k stands for an integer of from 1 to n. Concerning the position(s) of substitution, it is preferred to introduce the substituent(s) such that the resulting molecule has as high symmetry as possible. Described specifically, when n=3, it is more preferred to introduce the substituents to the 1,3,5-positions of a benzene ring rather than to its 1,2,4-positions.

The optical material making use of the aromatic sulfide compound according to the present invention can be used as a material for lenses or optical filters or as an antireflection film by combining it with a material of low refractive index into a laminated film. Further, the optical material can also be applied to lenses such as general camera lenses, video camera lenses, laser pickup lenses, fθ lenses for laser printers, Fresnel lenses, lenses for liquid crystal projectors, and eyeglass lenses; optical parts such as screens for projectors, optical fibers, optical waveguides, and prisms; and the like. Among these, it can be suitably used as a material for POFs.

Such optical parts can be divided into two groups, one containing a transparent polymer and a dopant in an evenly dispersed form, and the other containing them with a specific distribution. When an optical material has a refractive index distribution, it is preferred to apply the optical material to array lenses for use in GI POFs and copying machines.

For the production of the optical material according to the present invention, conventionally known molding processes can be used including injection molding, compression molding, micromolding, floating molding, the Rolinx process, and casting. According to casting, an optical material according to the present invention may be produced as a molded product by allowing polymerization of the aromatic sulfide compound to proceed partially, pouring the partially-polymerized aromatic sulfide compound into a mold, and then subjecting it to final polymerization. According to injection molding, on the other hand, a molding sample can be obtained by adding a dopant to a thermoplastic ' resin and stirring them until a homogeneous mixture is formed. According to pouring, an optical material can be obtained, for example, by adding a dopant to a UV curable monomer and mixing them until a homogeneous mixture is formed.

Molded products obtained by such molding processes as described above can be improved in moisture resistance, optical properties, chemical resistance, abrasion resistance, anti-mist property and the like by coating their surfaces with an inorganic compound such as MgF₂ or SiO₂ in accordance with vacuum deposition, sputtering, ion plating or the like or by applying an organic silicon compound such as a silane coupling agent, a vinyl monomer, a melamine resin, an epoxy resin, a fluorinated resin, a silicone resin or the like as hard coatings onto their surfaces.

A description will hereinafter be made more specifically about use of the aromatic sulfide compound according to the present invention as a material for GI POFs.

When the aromatic sulfide compound according to the present invention is used for such an application, it is generally used as a high refractive index dopant. The refractive index may preferably be in a range of from 1.60 to 2.0, with a range of 1.63 to 1.90 being more preferred.

One of these high refractive index dopants may be singly included in a core part, or plural ones of such dopants may be chosen and included in combination in a core part. As a further alternative, one or more of these dopants may be included along with one or more other known dopants in a core part.

No particular limitation is imposed on the content of the high refractive index dopant in the core part of POF insofar as a desired refractive index distribution is obtained and the fiber is not impaired in mechanical strength and the like. Upon production of a POF material by polymerization, it is preferred to have the high refractive index dopant included in the core part of the produced POF material by adding the dopant to a monomer for a core-part-forming polymer and then subjecting the resultant mixture of the monomer and the dopant to a polymerization reaction. The content of the high refractive index dopant in the core part of POF may be preferably 60 wt.% or lower, more preferably 50 wt.% or lower, still more preferably 45 wt.% or lower.

No particular limitation is imposed on the molecular volume of the high refractive index dopant compound according to the present invention useful in POFs, because the molecular volume is determined depending upon the monomer for the core-part POF material to be used in combination with the dopant compound. In' view of the fact that the molecular volume of methyl methacrylate employed in conventional POFs is approximately 101 Å³, the molecular volume is preferably in a range of from 100 to 500 Å³, more preferably in a range of from 150 to 400 Å³, both when methyl methacrylate is used as a core-part preform monomer.

Existence of a large difference in refractive index between a central part and an outer peripheral part of an optical fiber is preferred because such a large difference makes it possible not only to lower the transmission loss but also to reduce the connection loss and bending loss. The numerical aperture of a plastic optical fiber according to the present invention may preferably in a range of from 0.15 to 0.40, more preferably in a range of from 0.18 to 0.30.

As has been described above, aromatic sulfide compounds according to the present invention comprise compounds having the skeleton represented by formula (1). Specific examples of such aromatic sulfide compounds can include the compounds described in the following table.

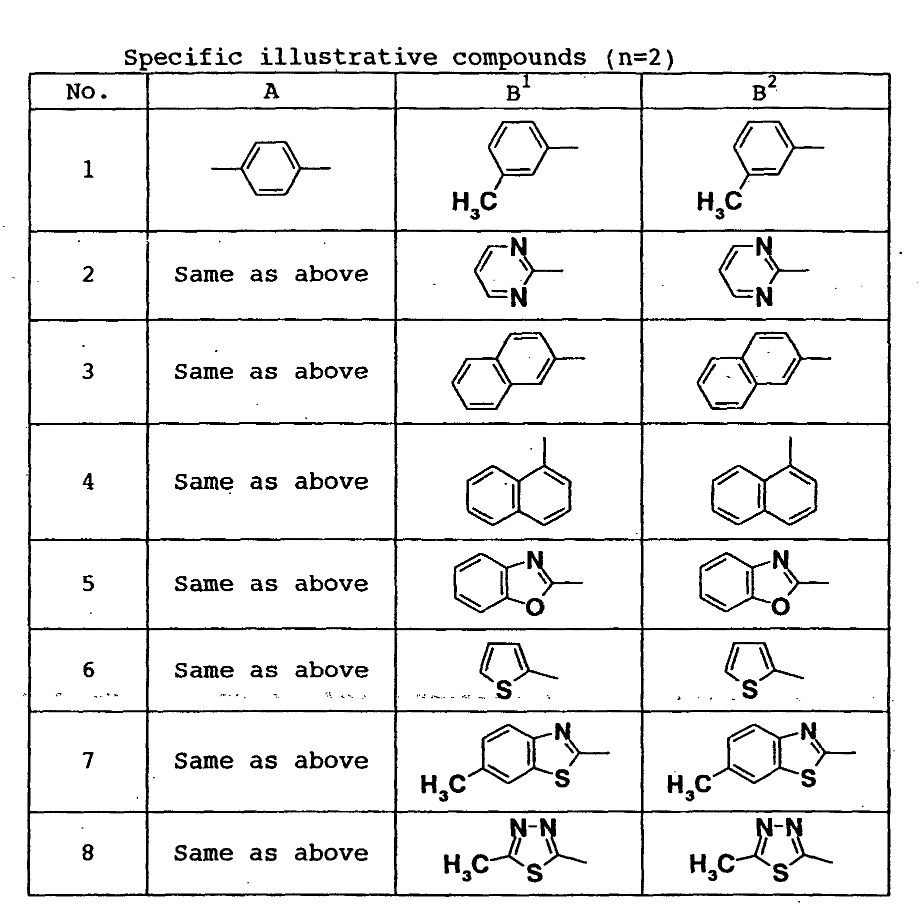

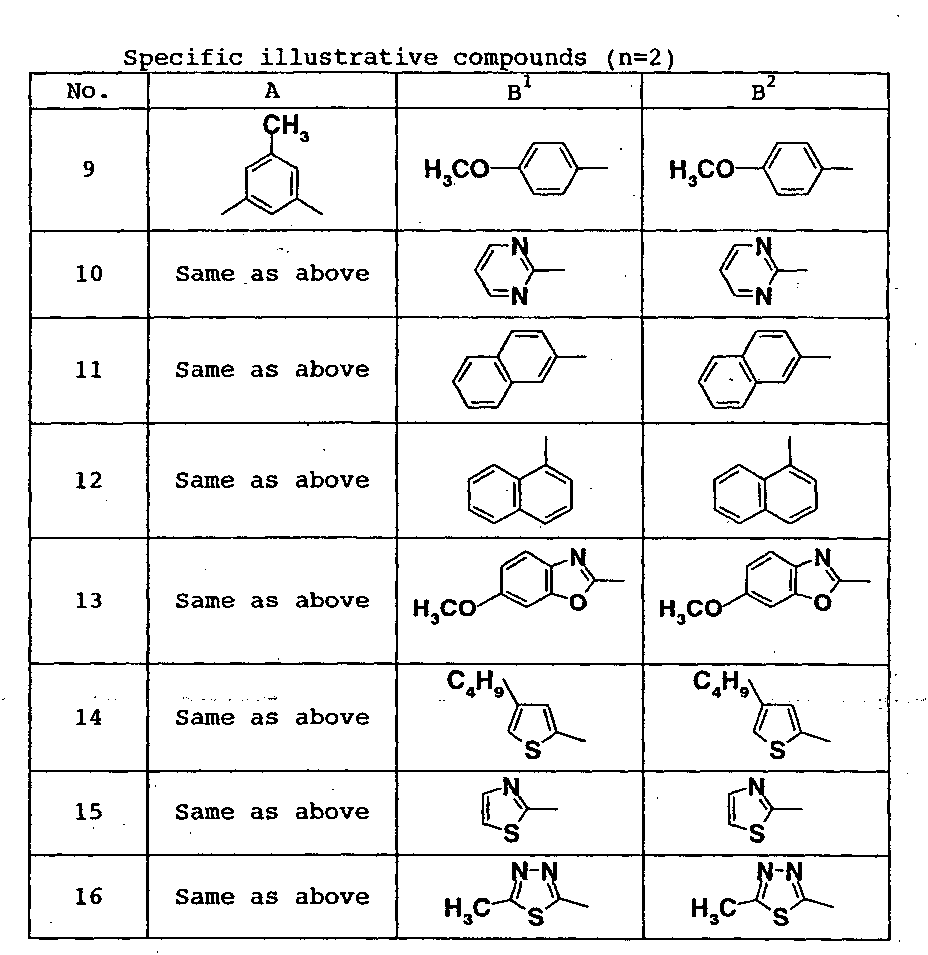

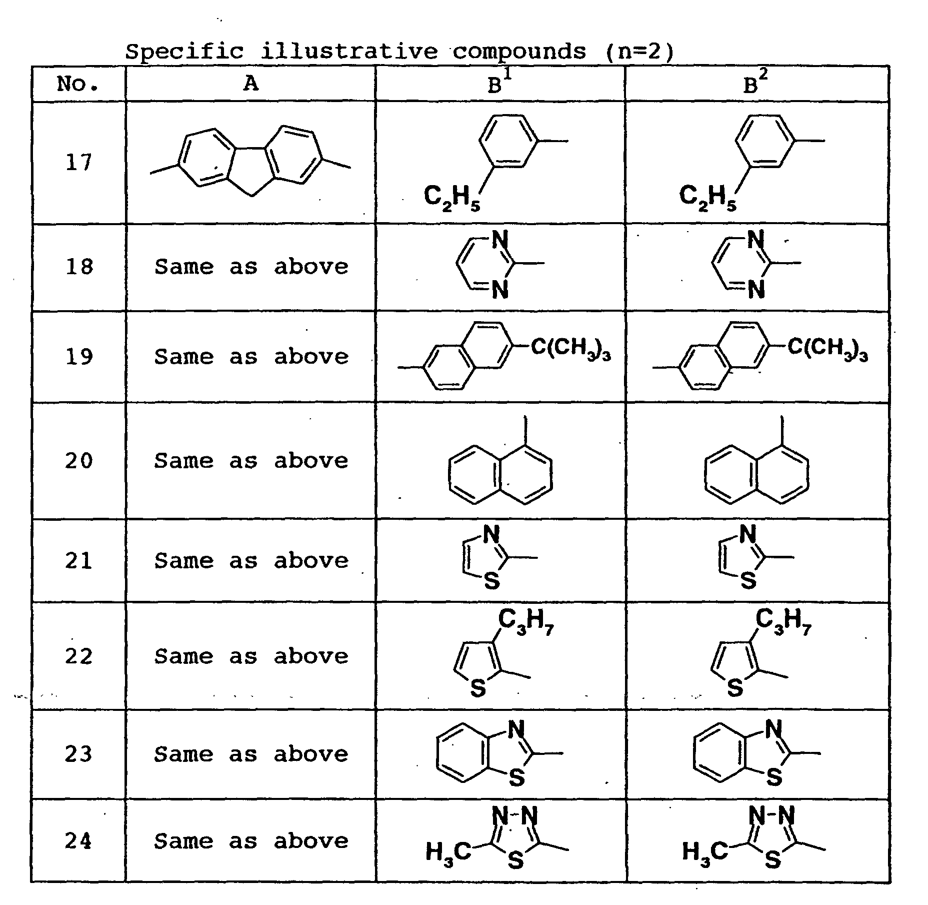

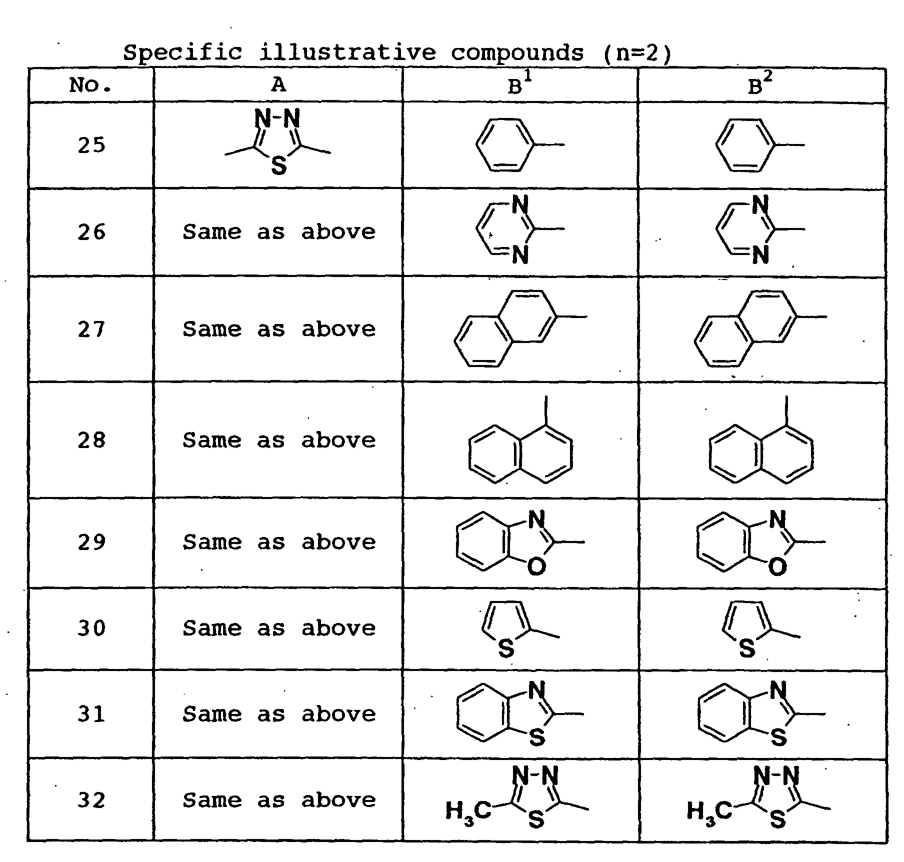

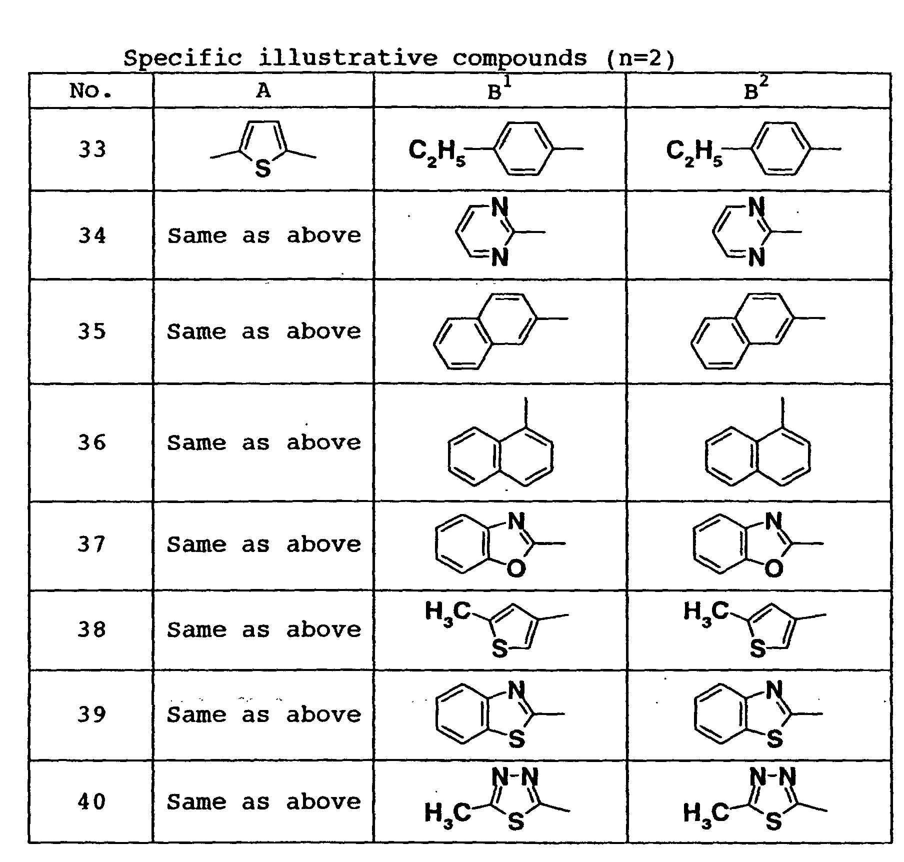

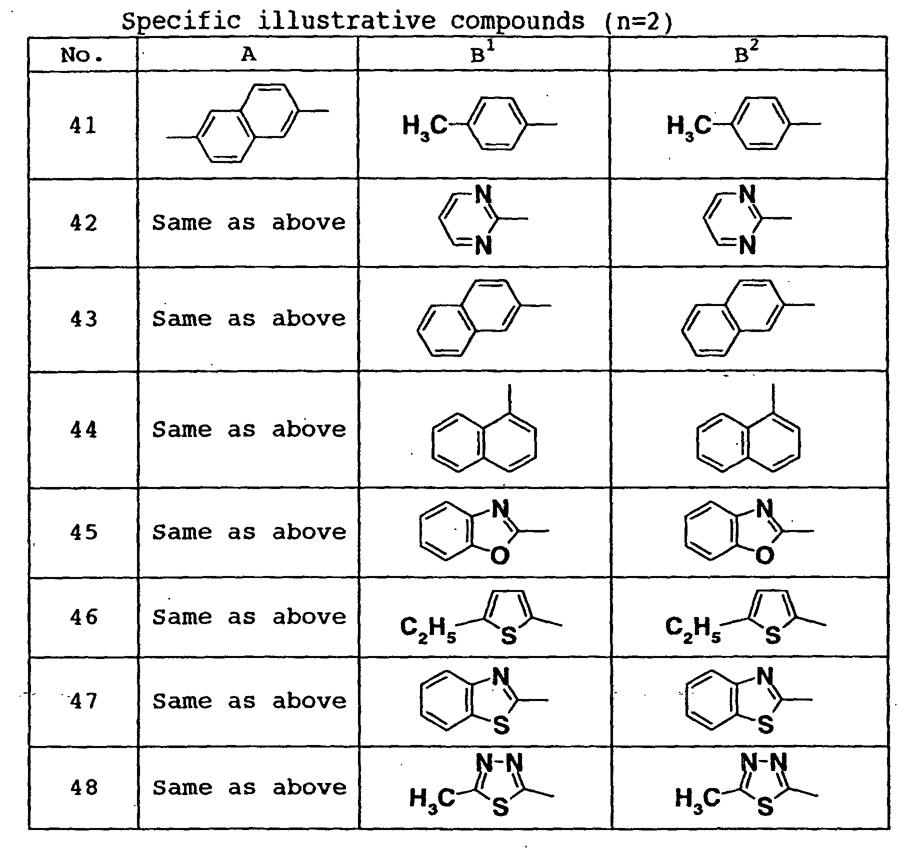

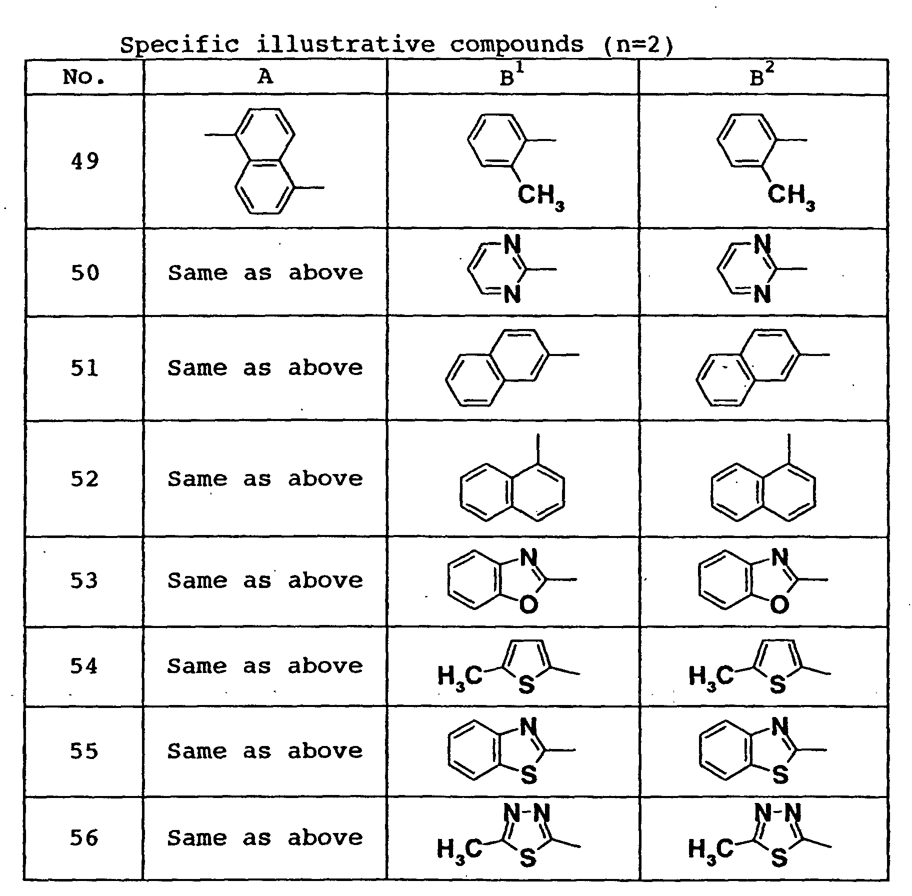

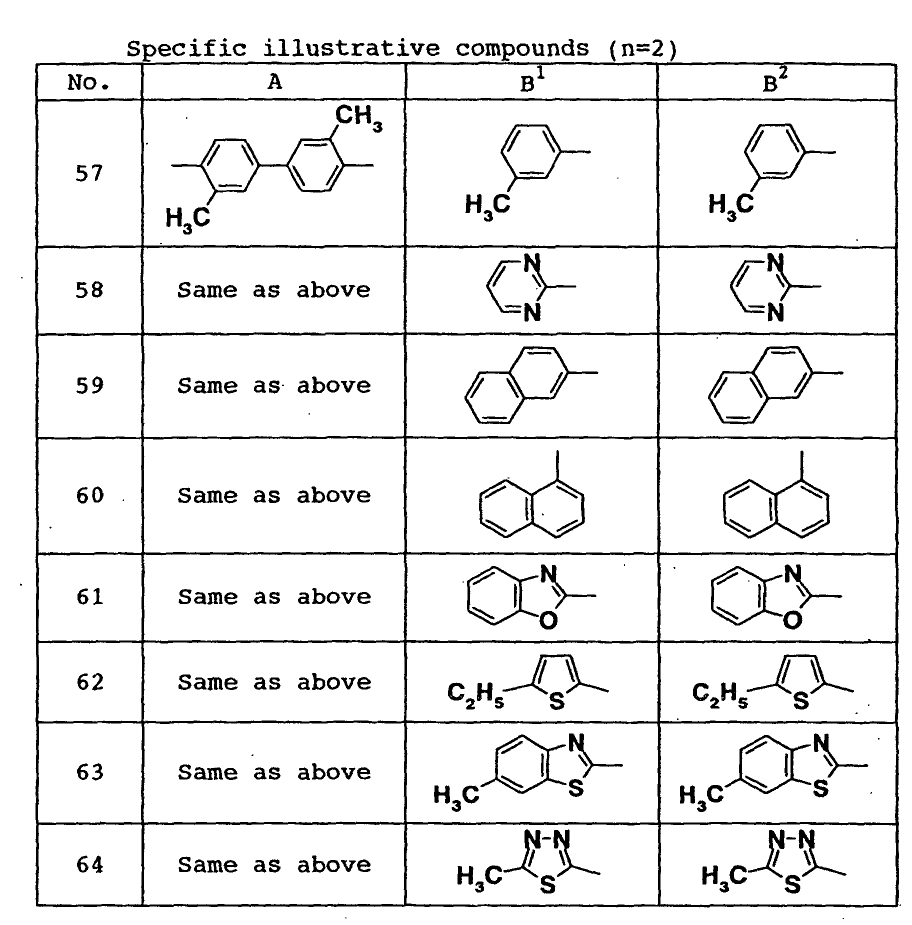

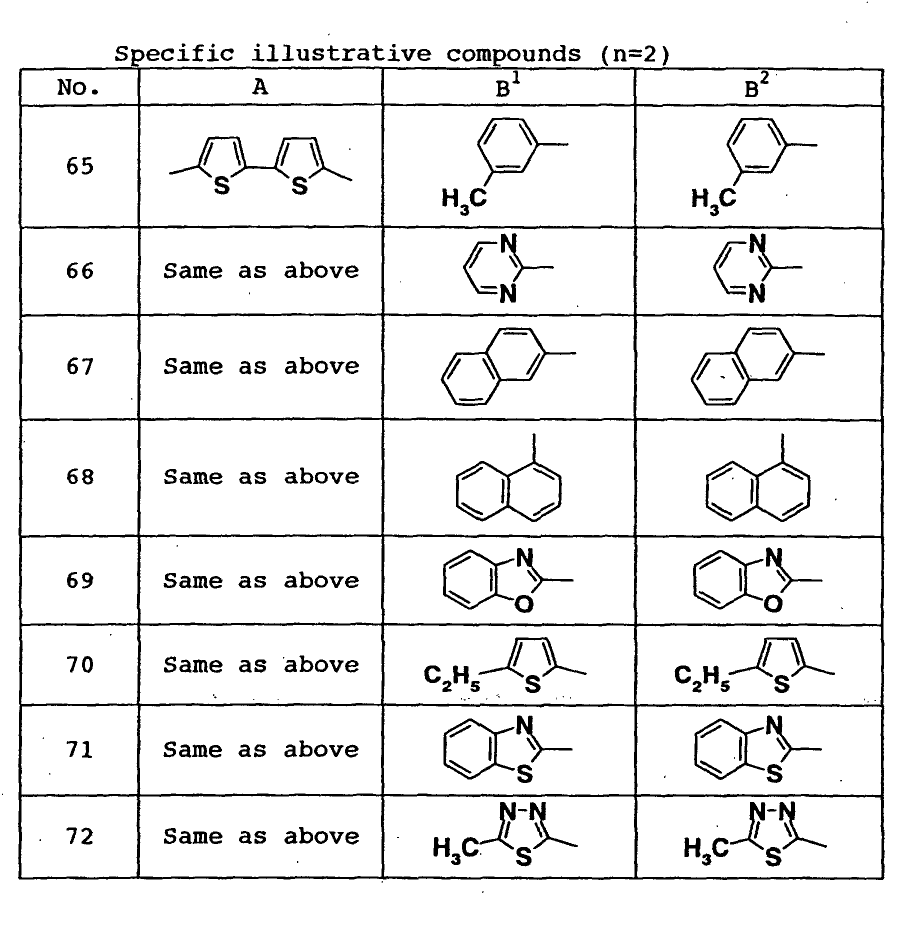

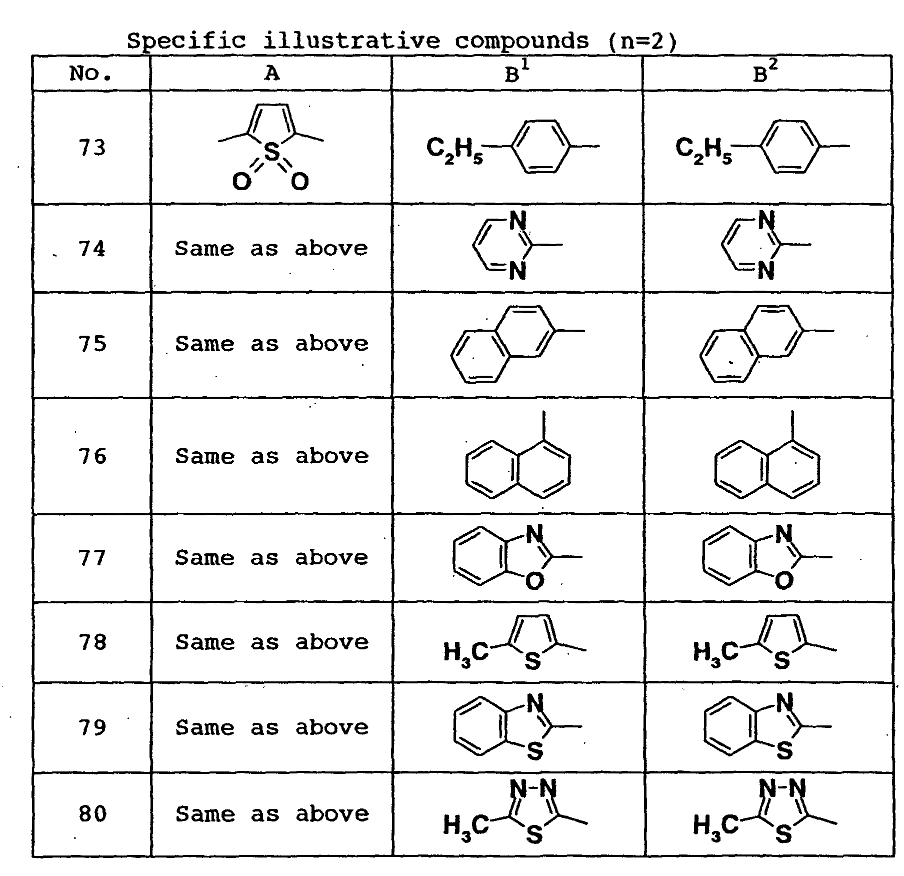

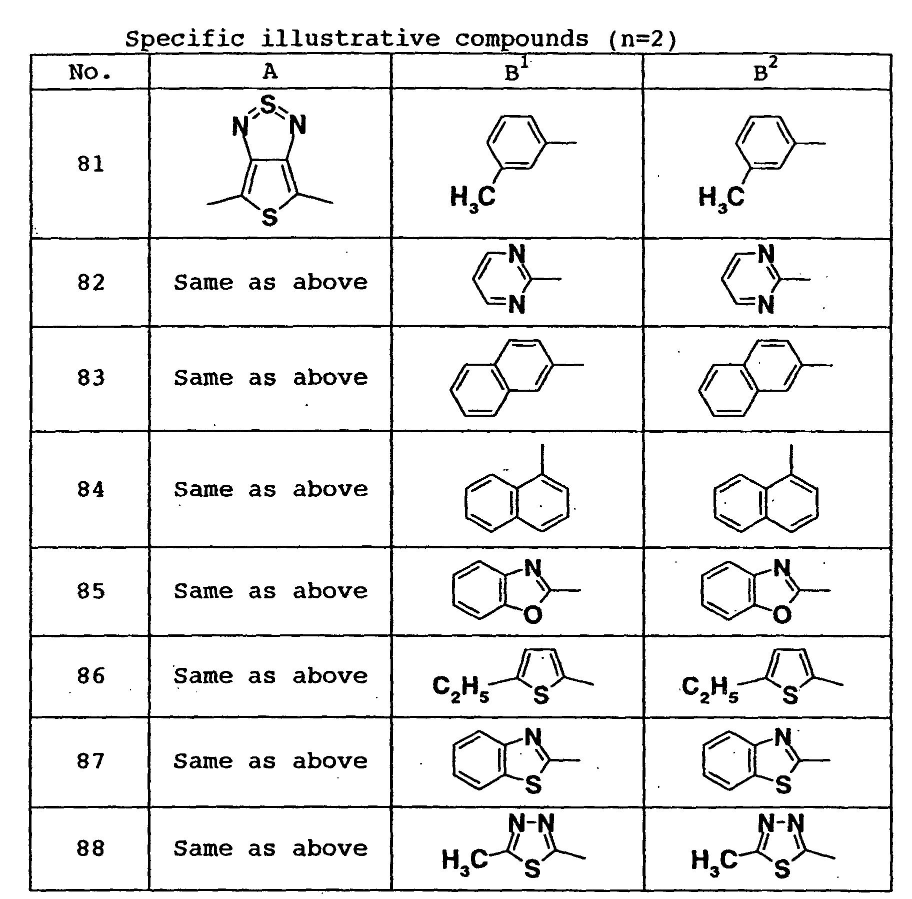

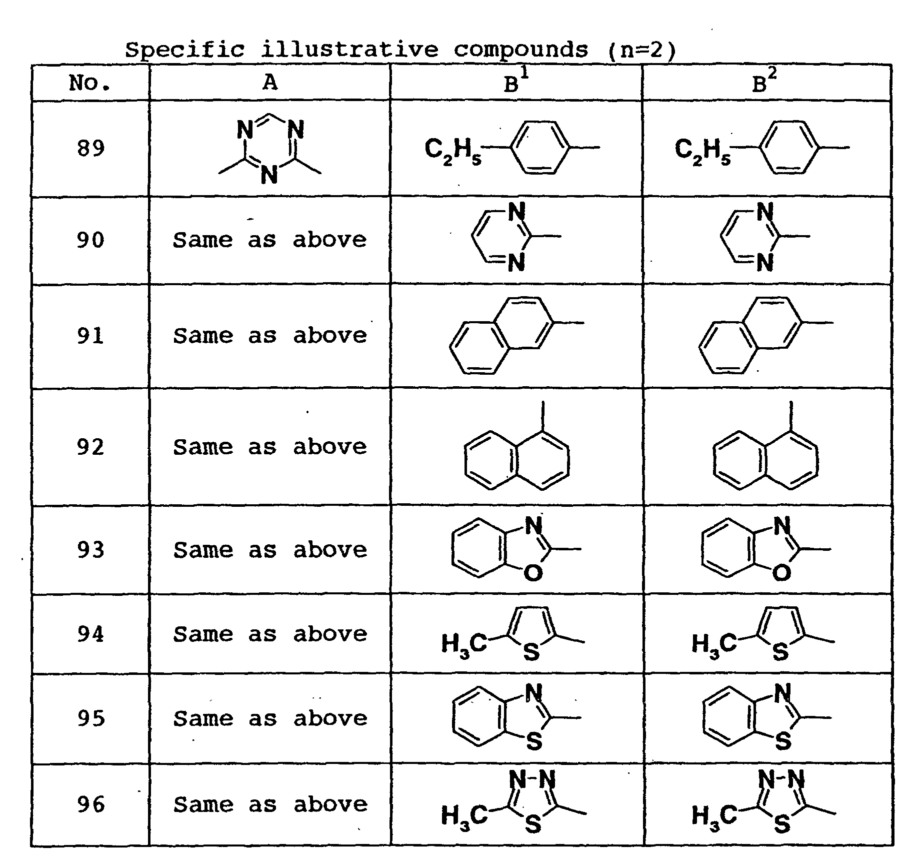

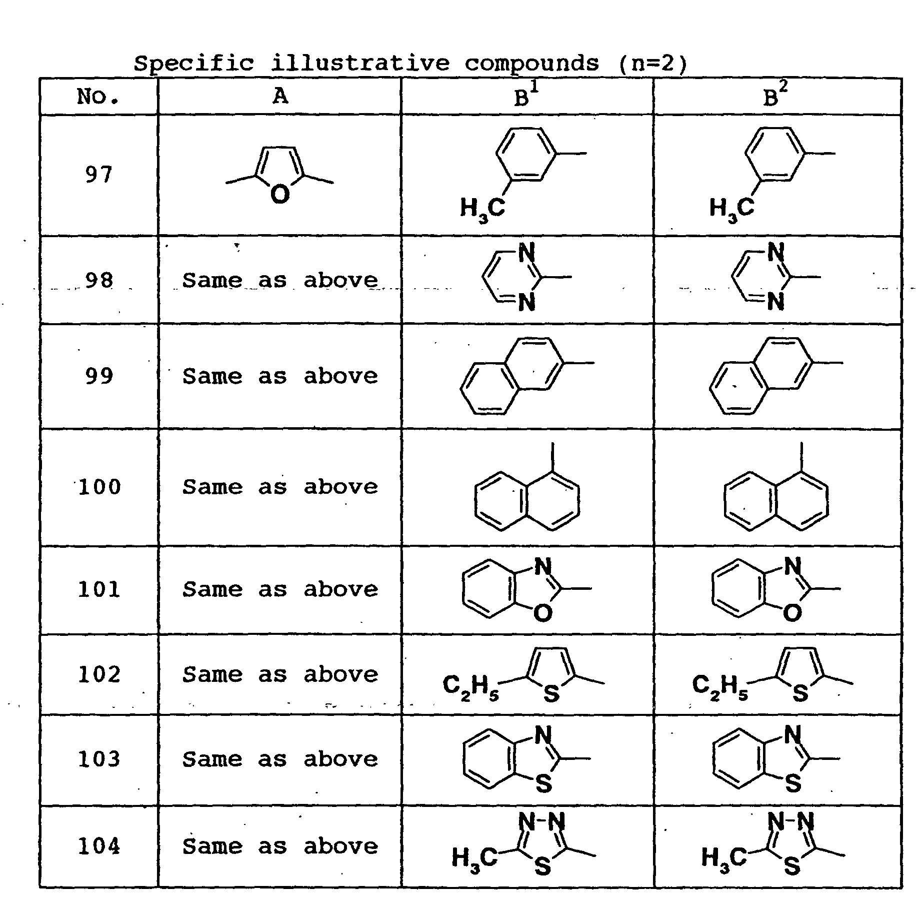

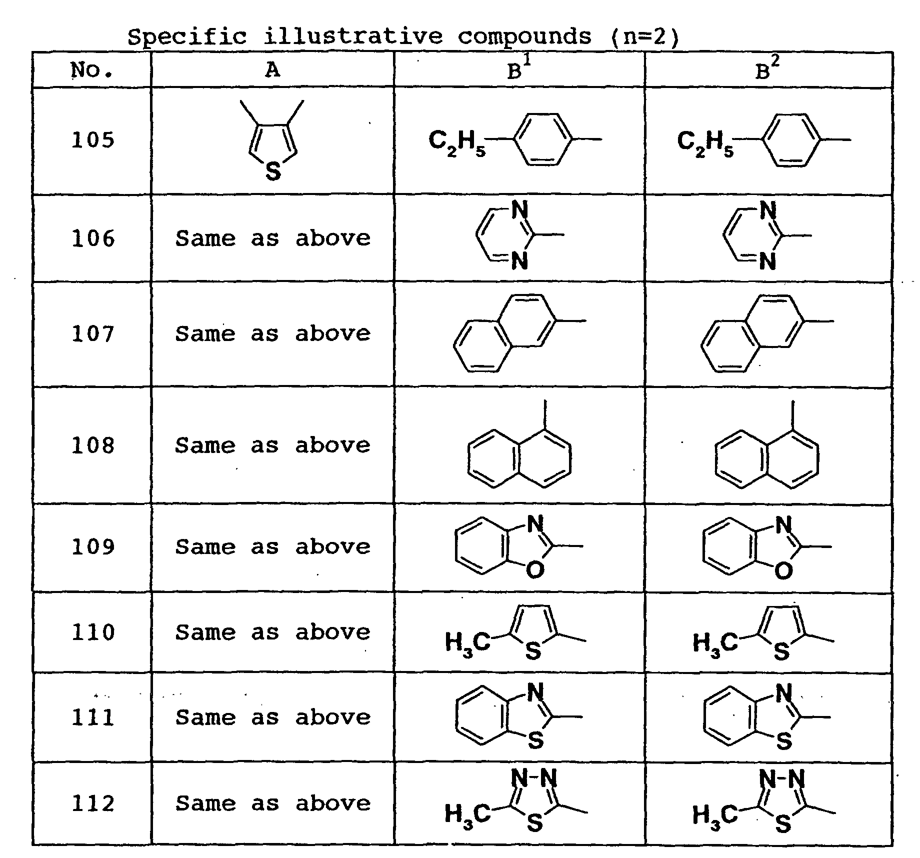

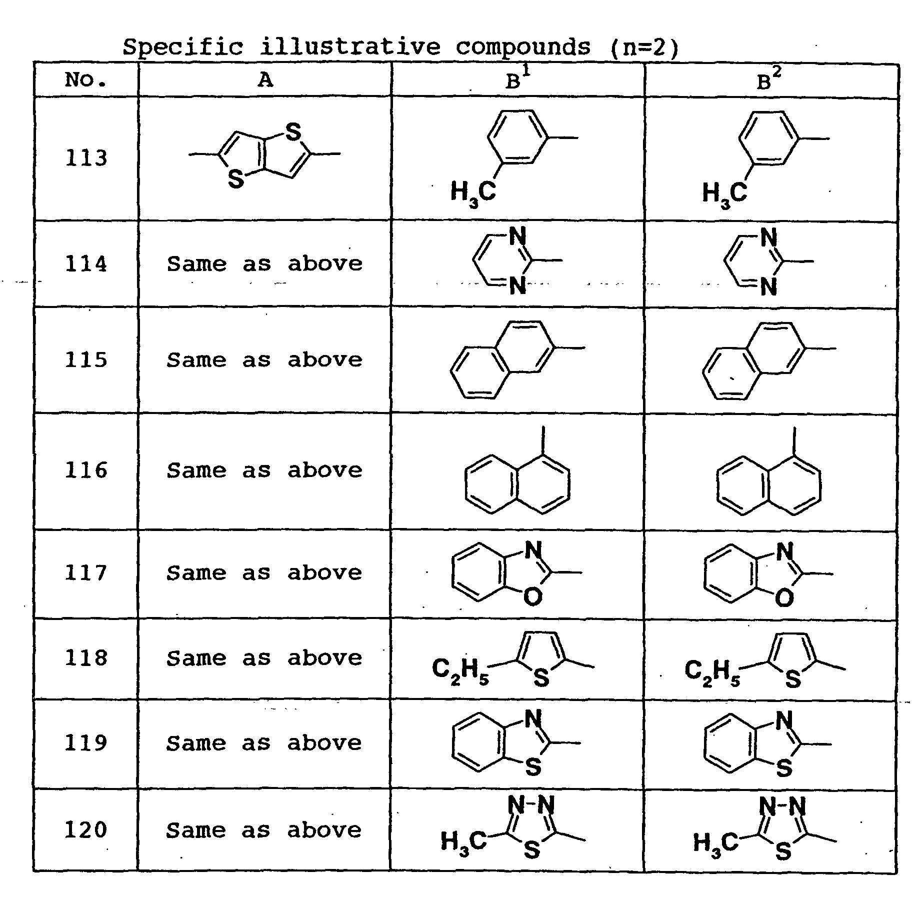

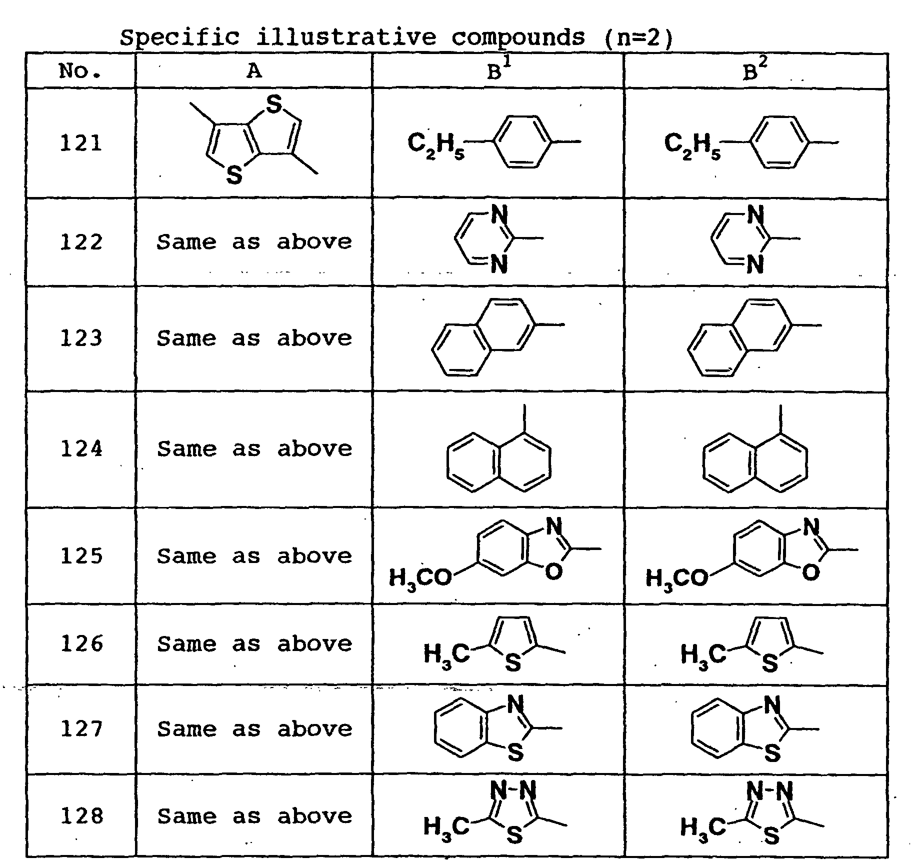

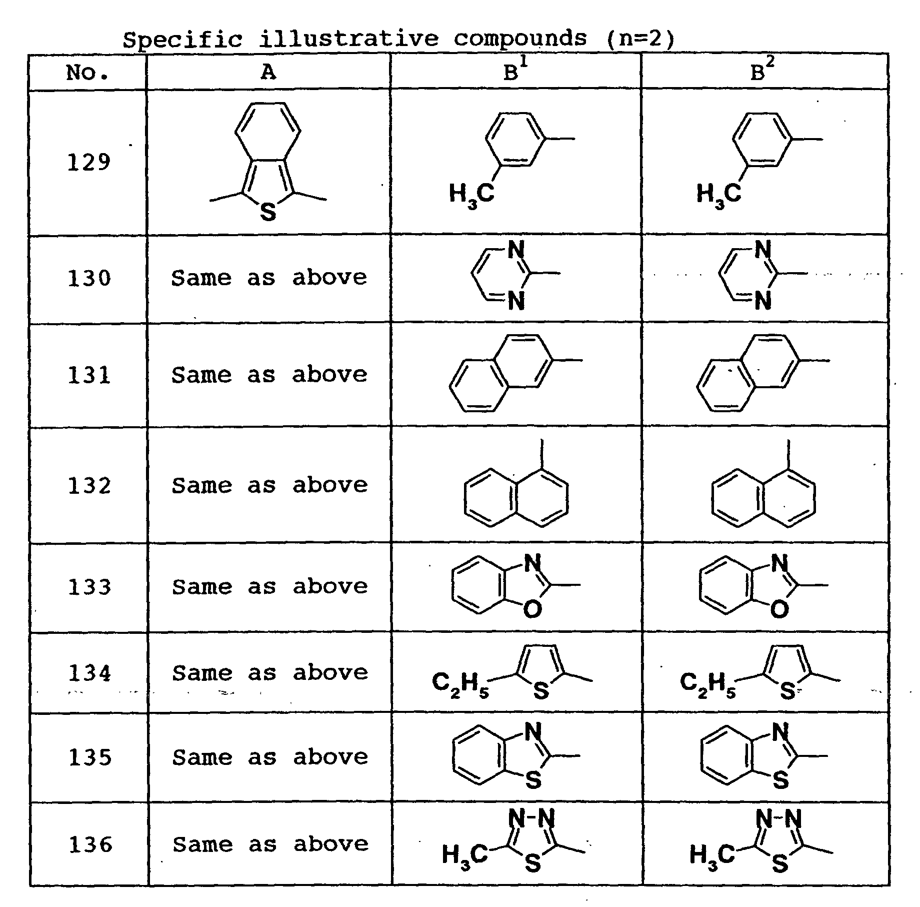

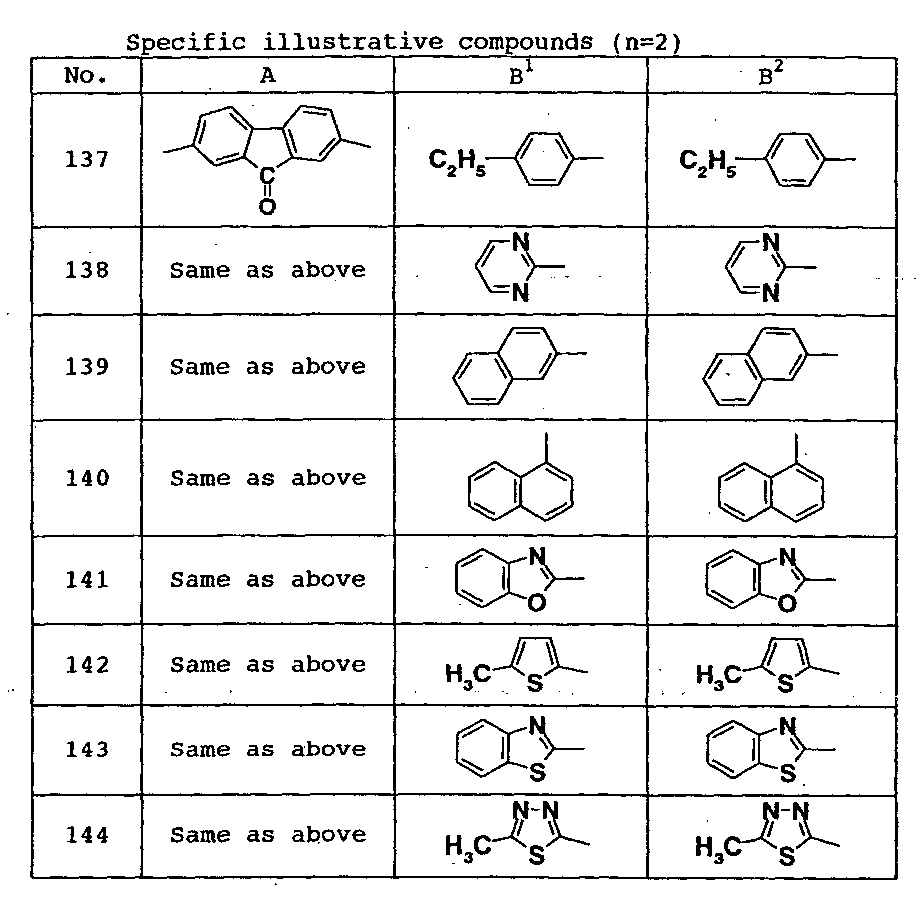

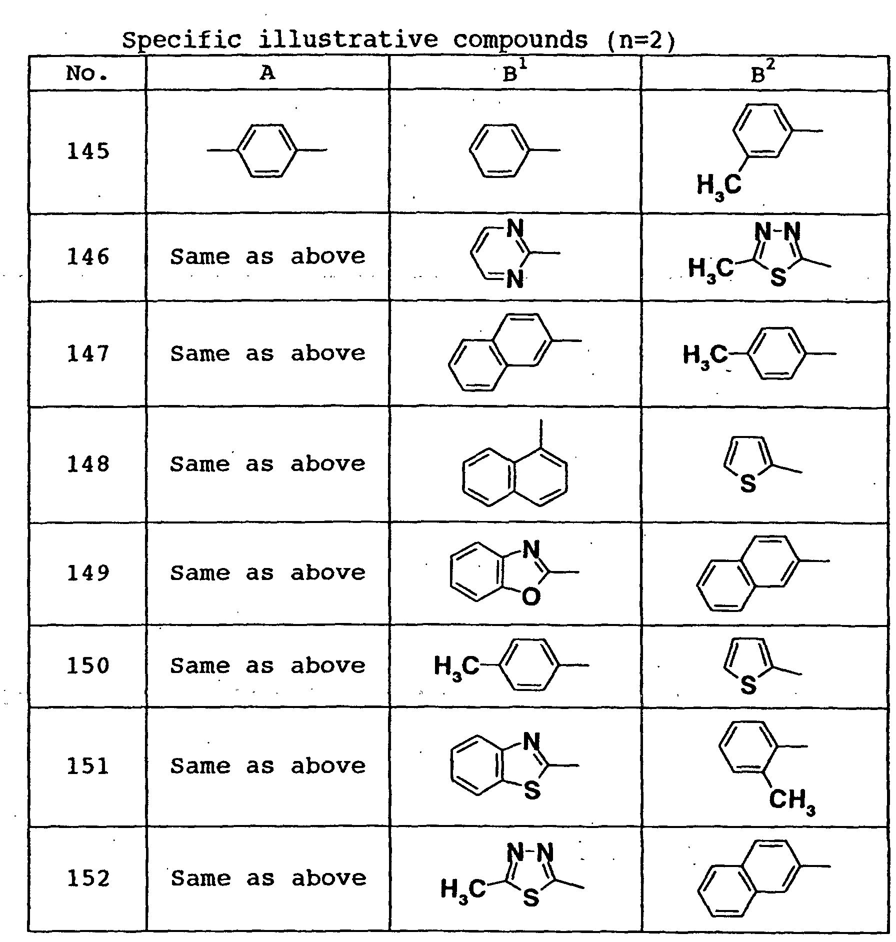

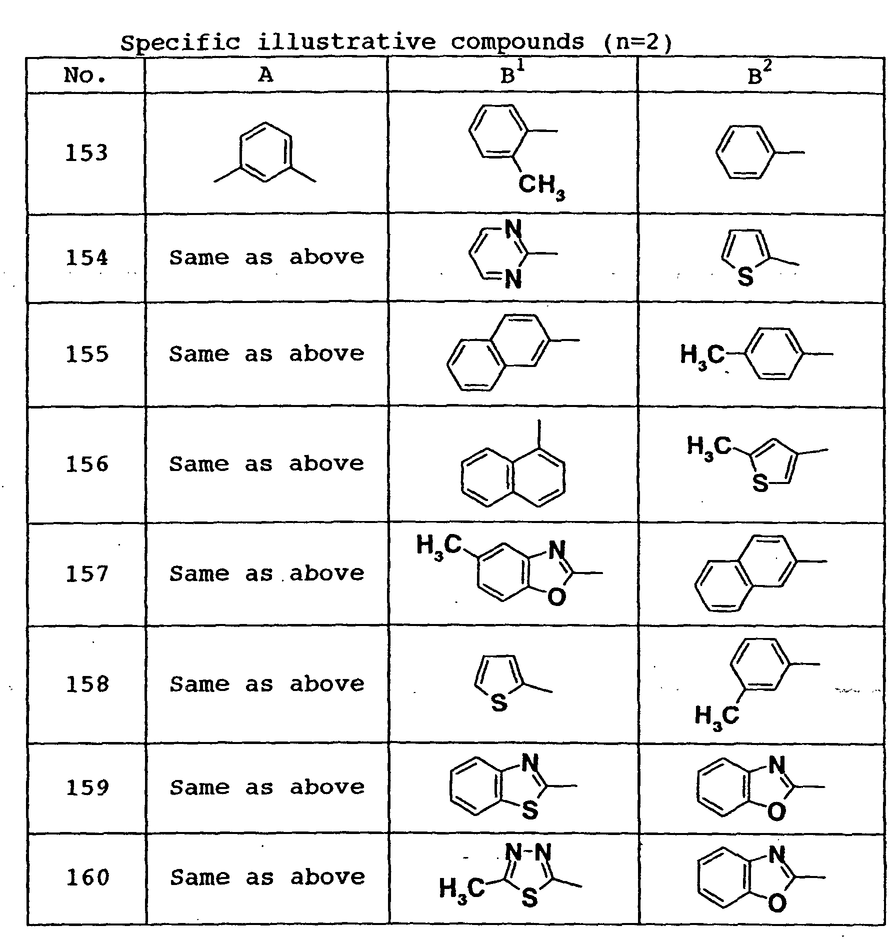

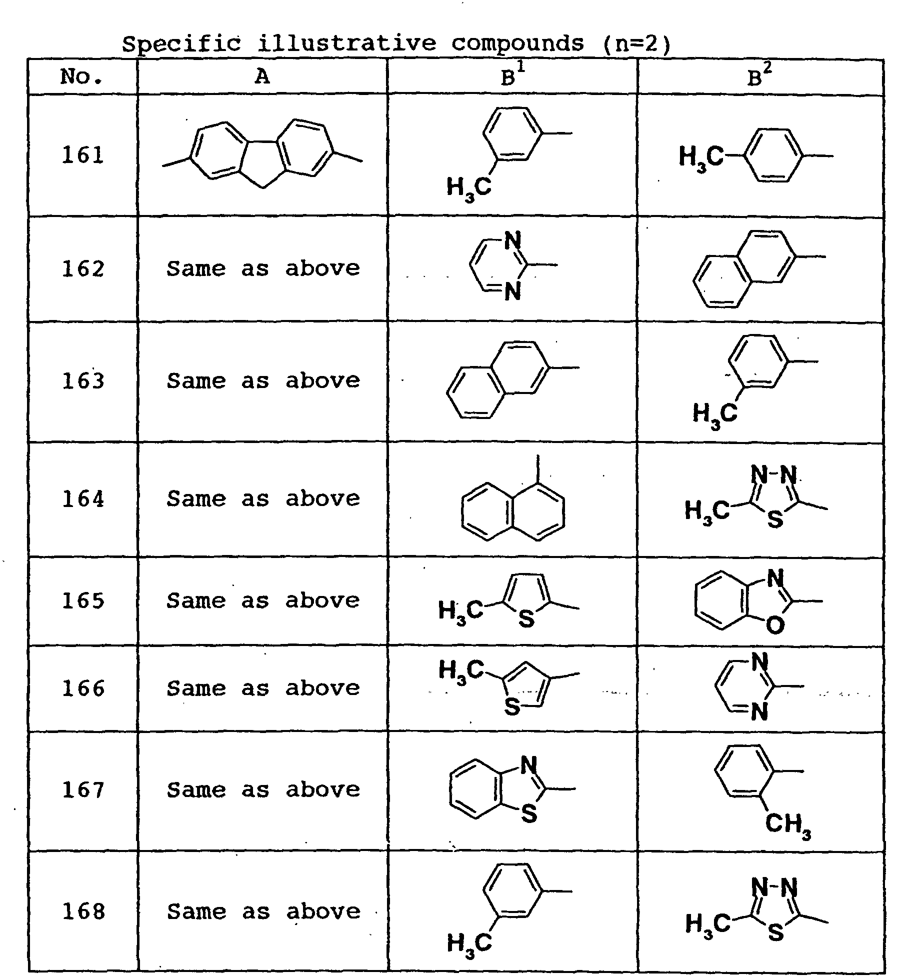

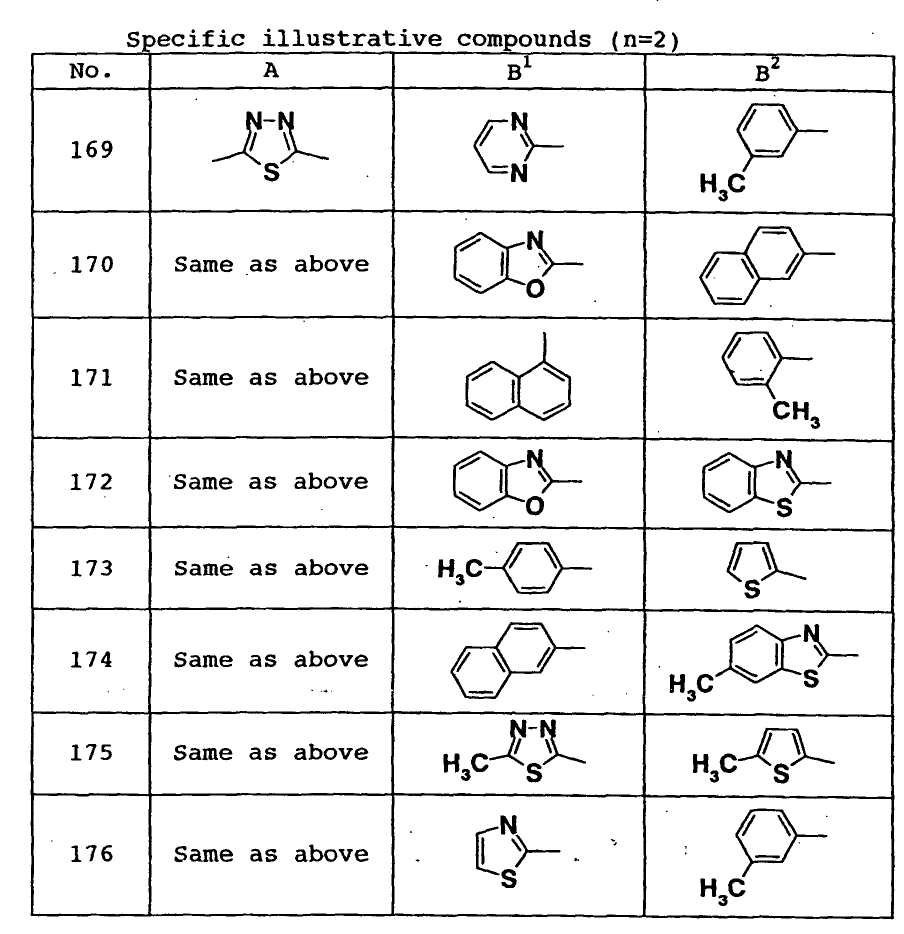

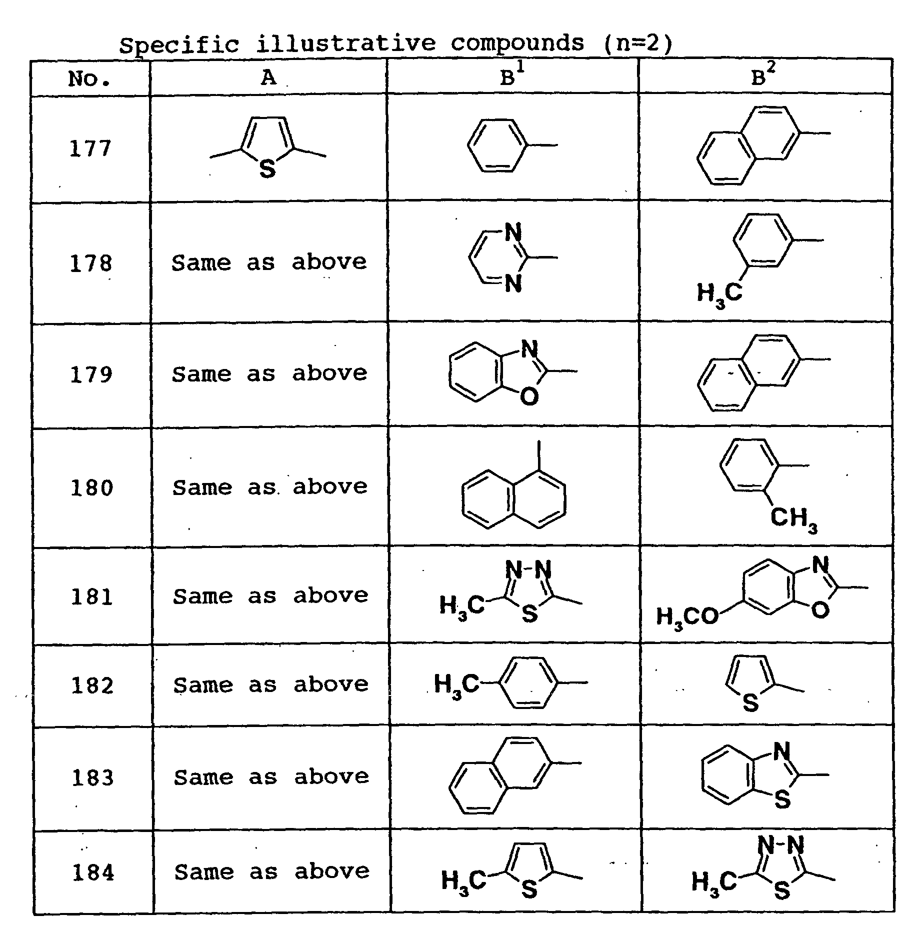

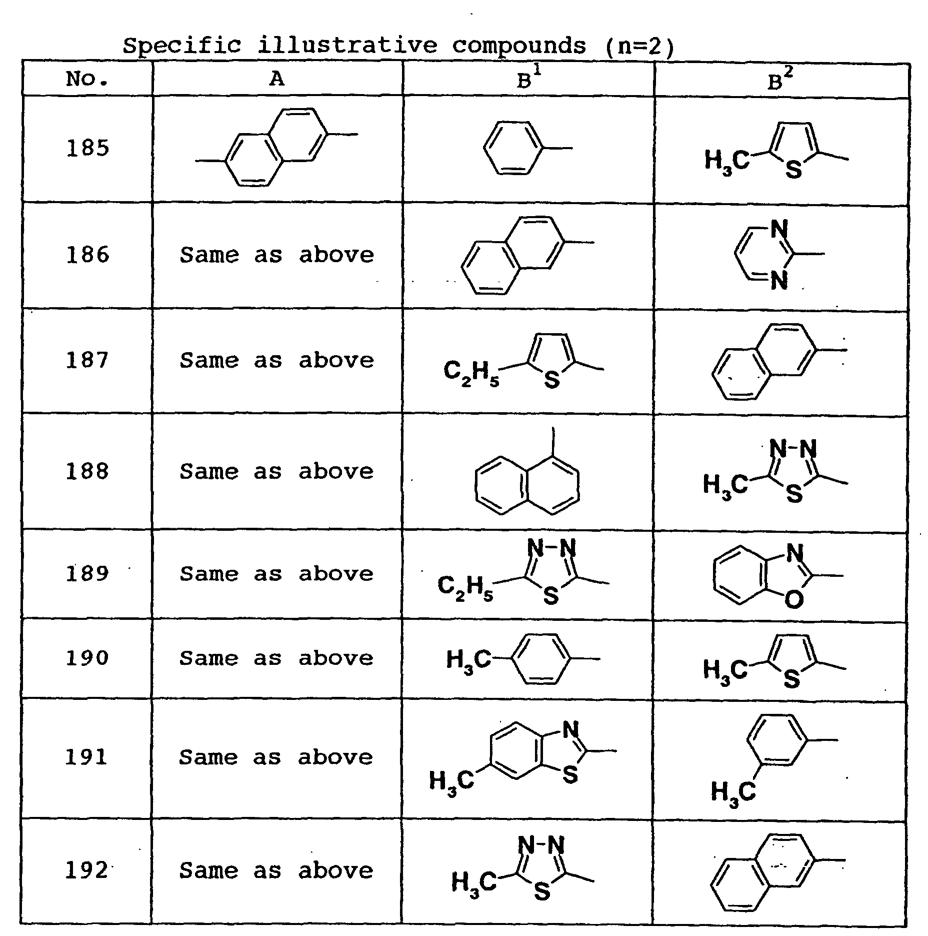

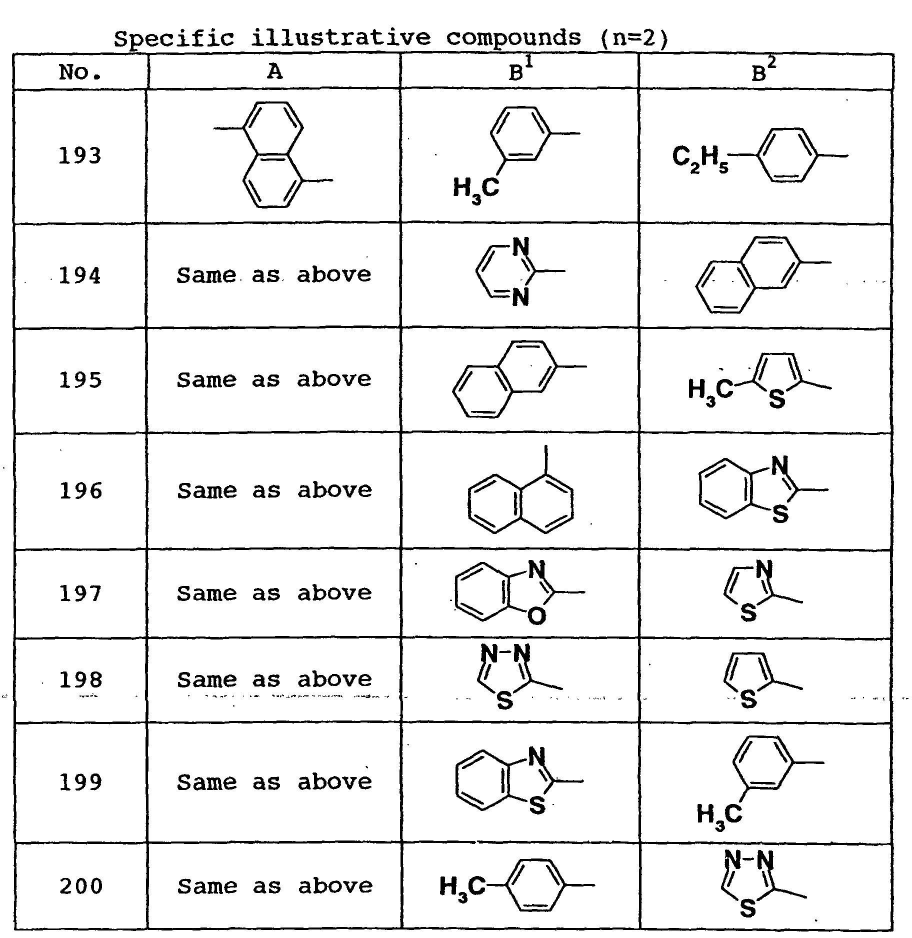

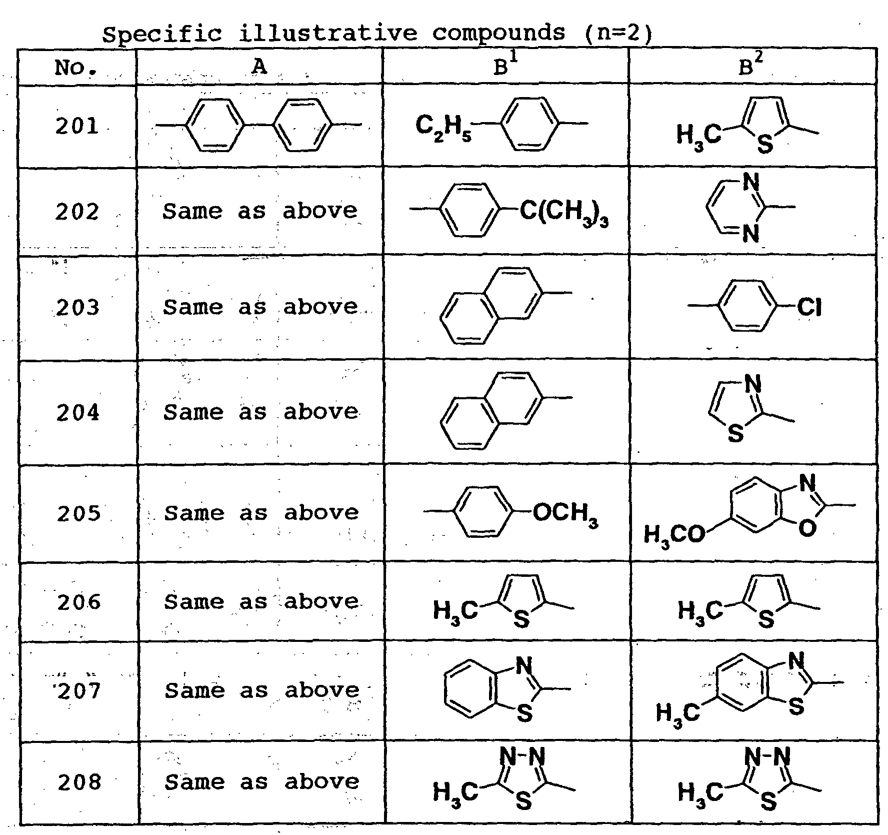

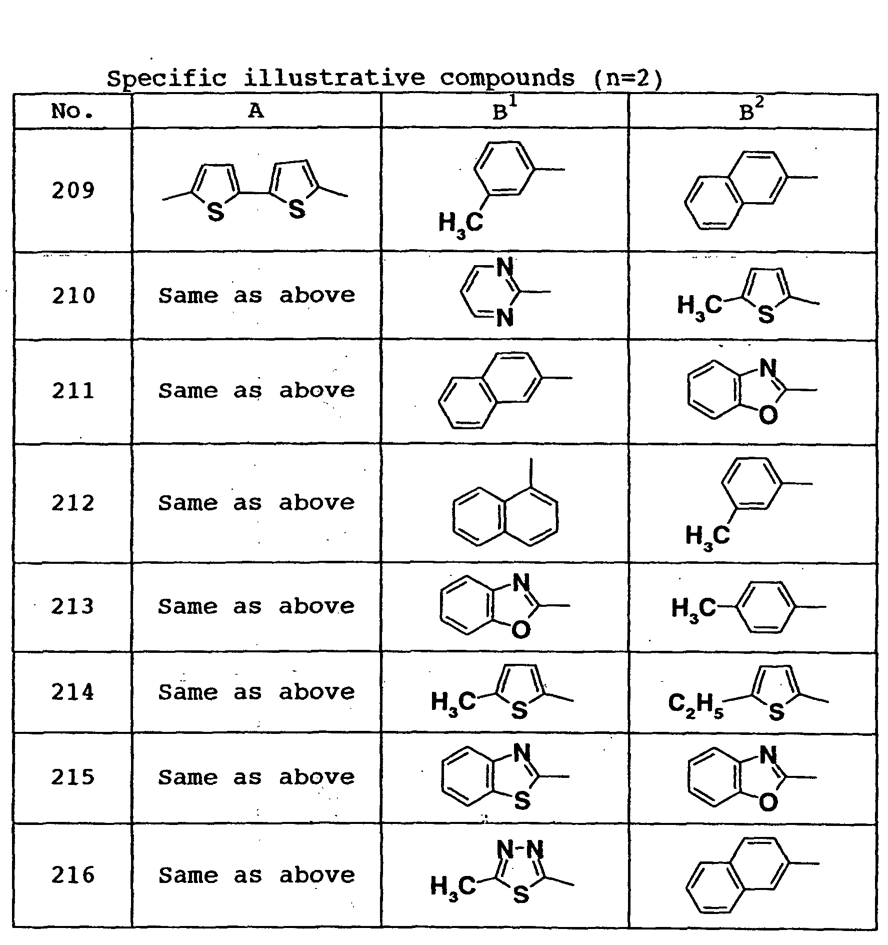

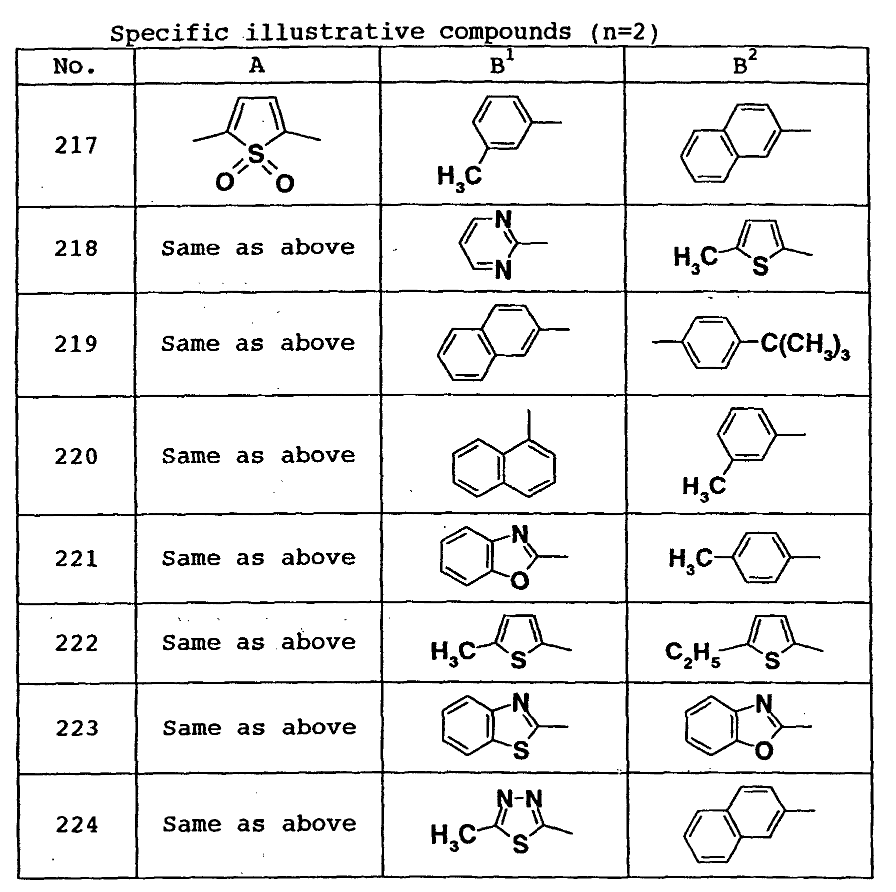

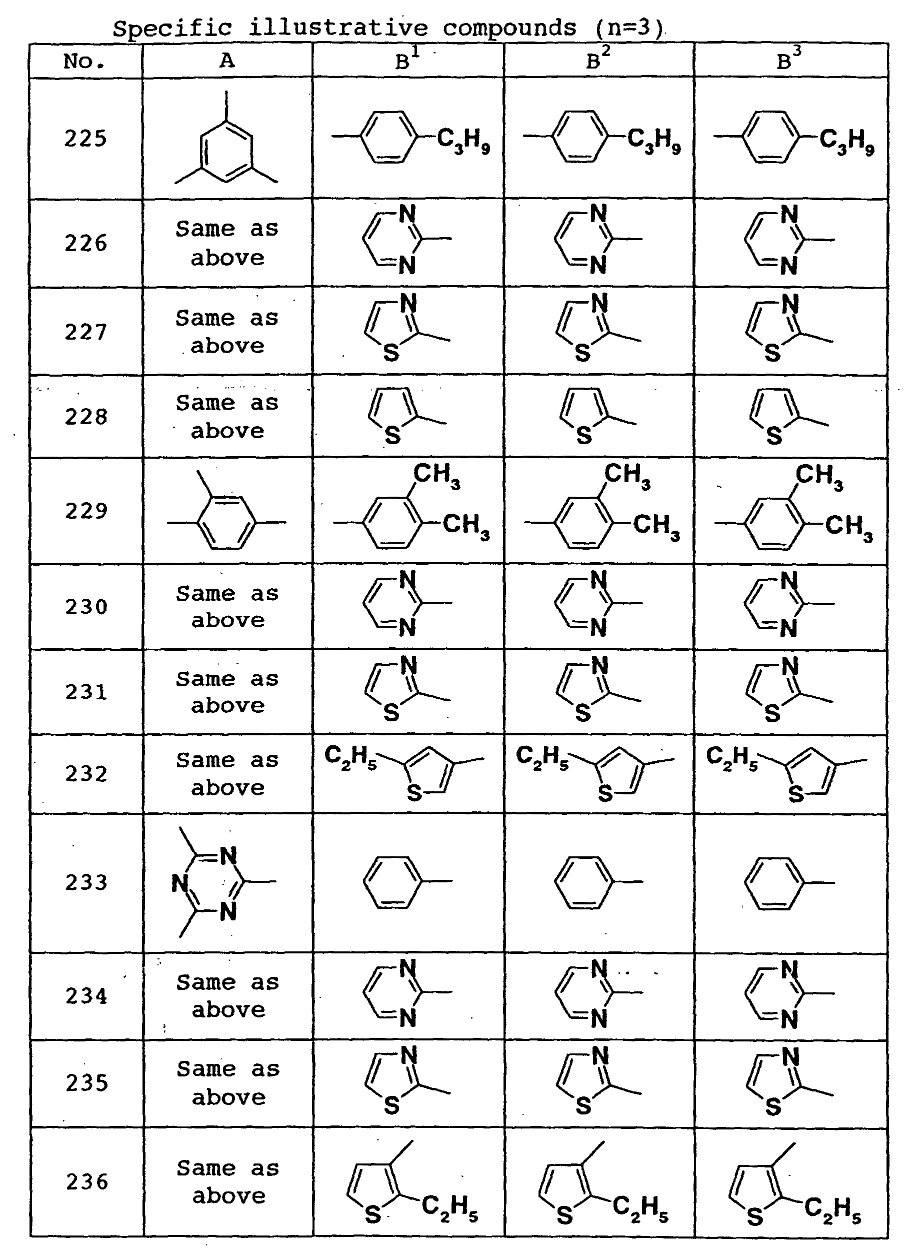

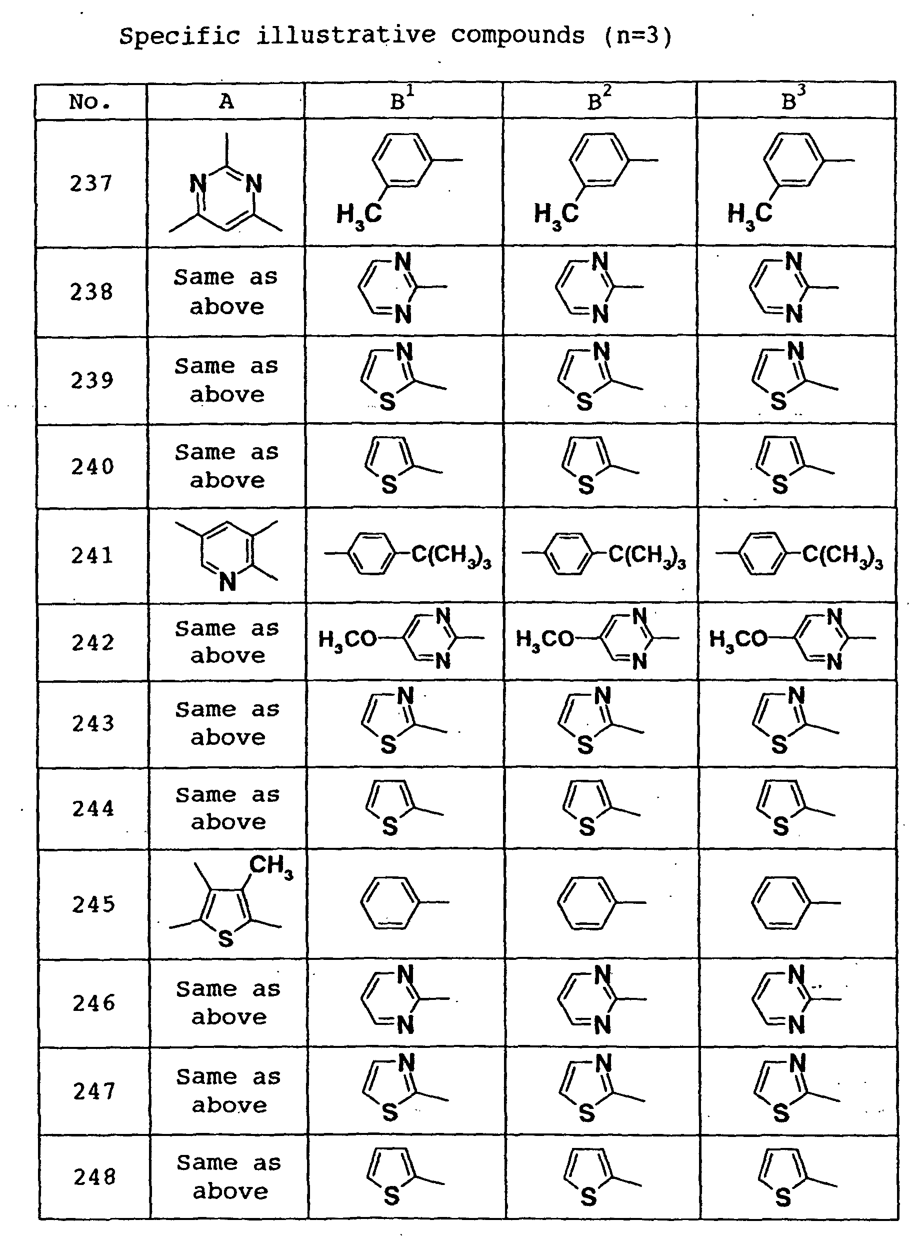

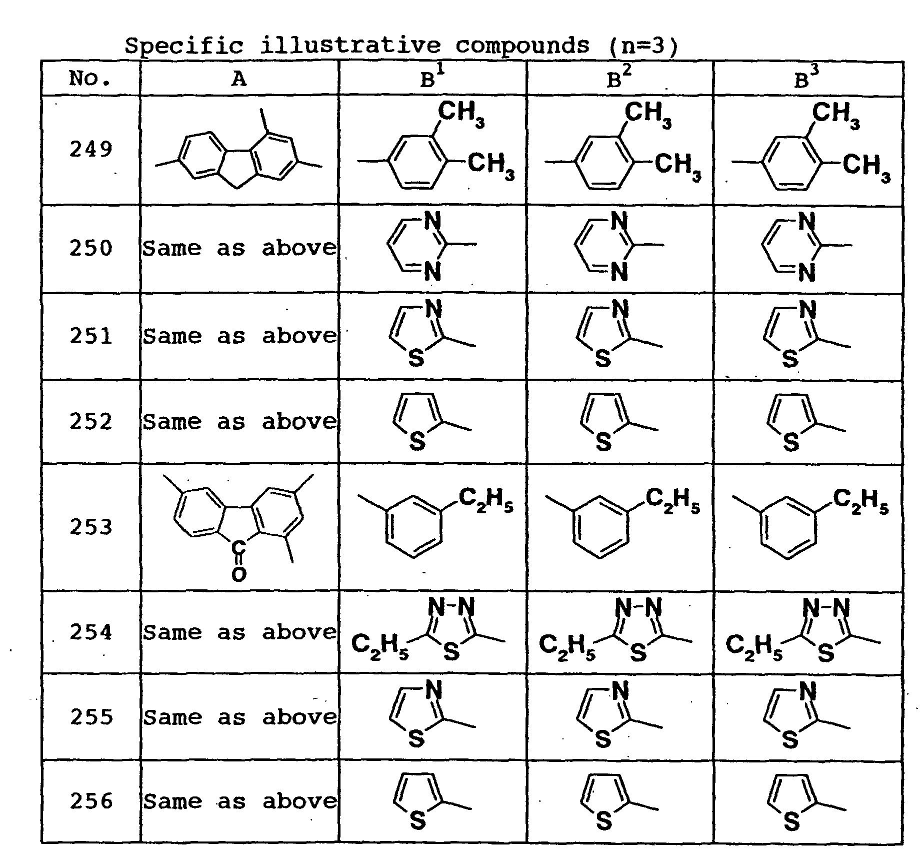

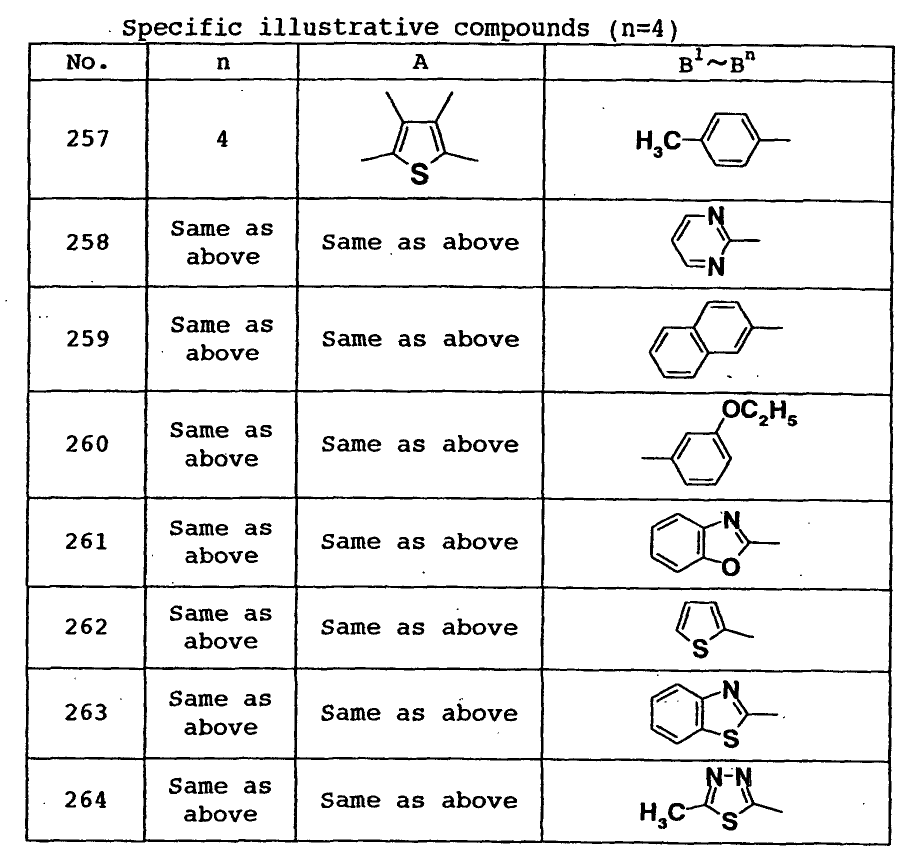

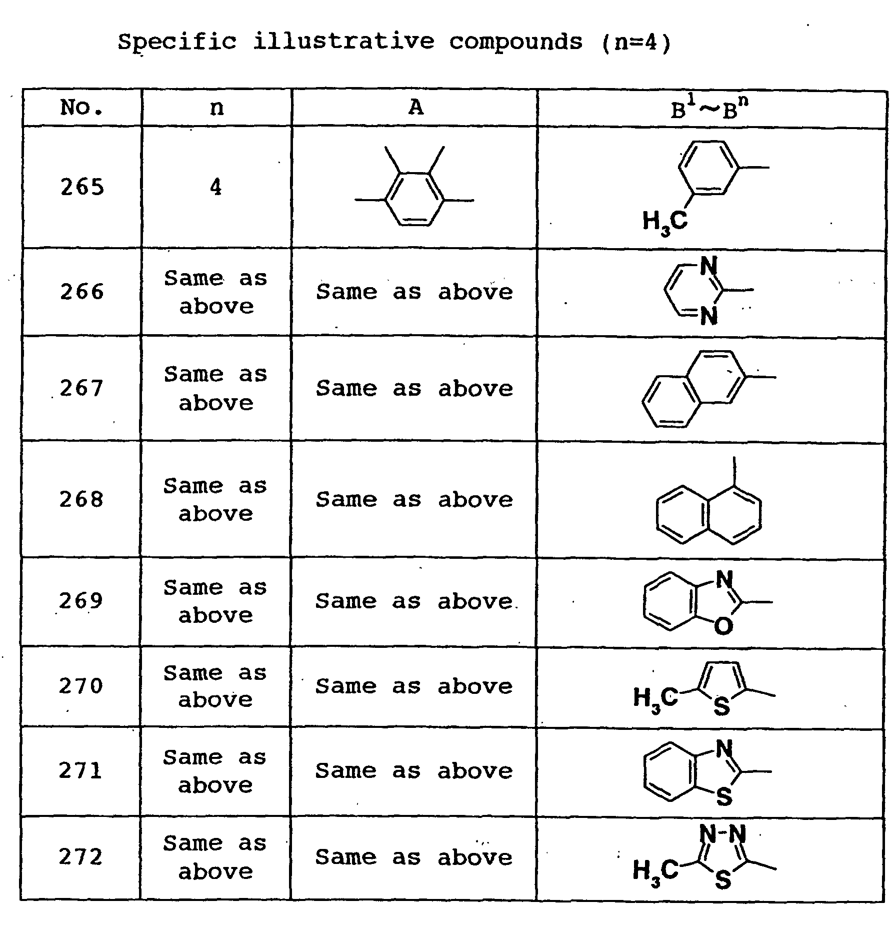

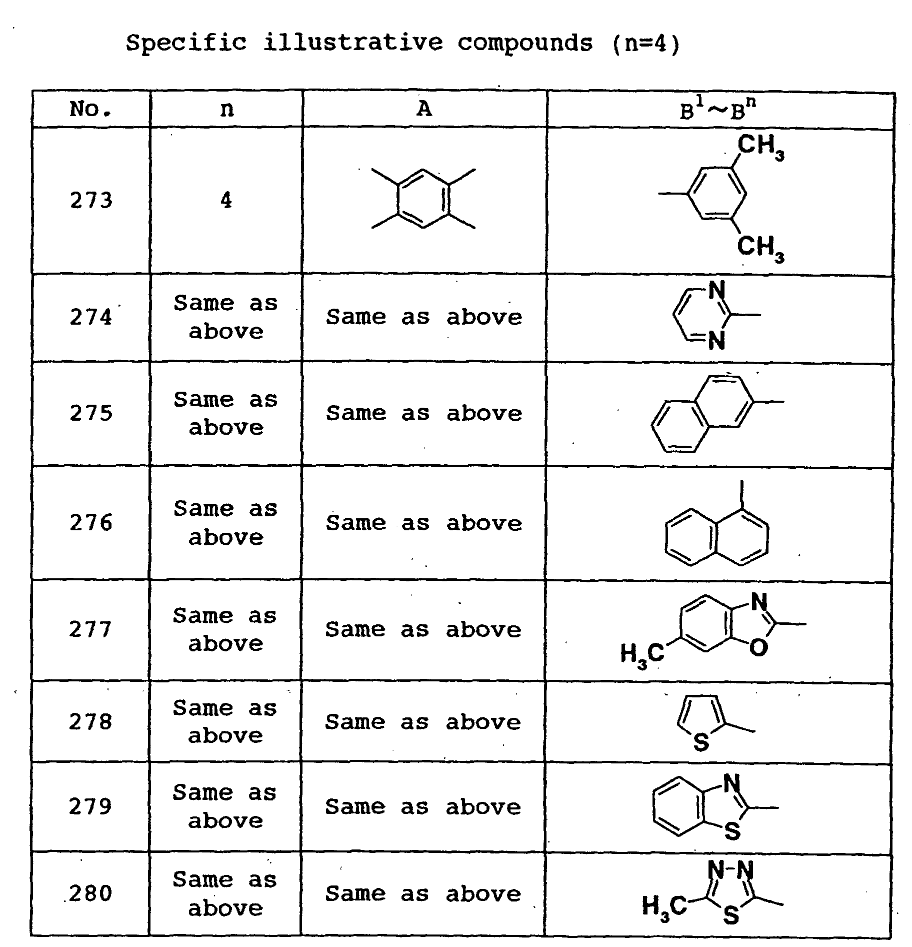

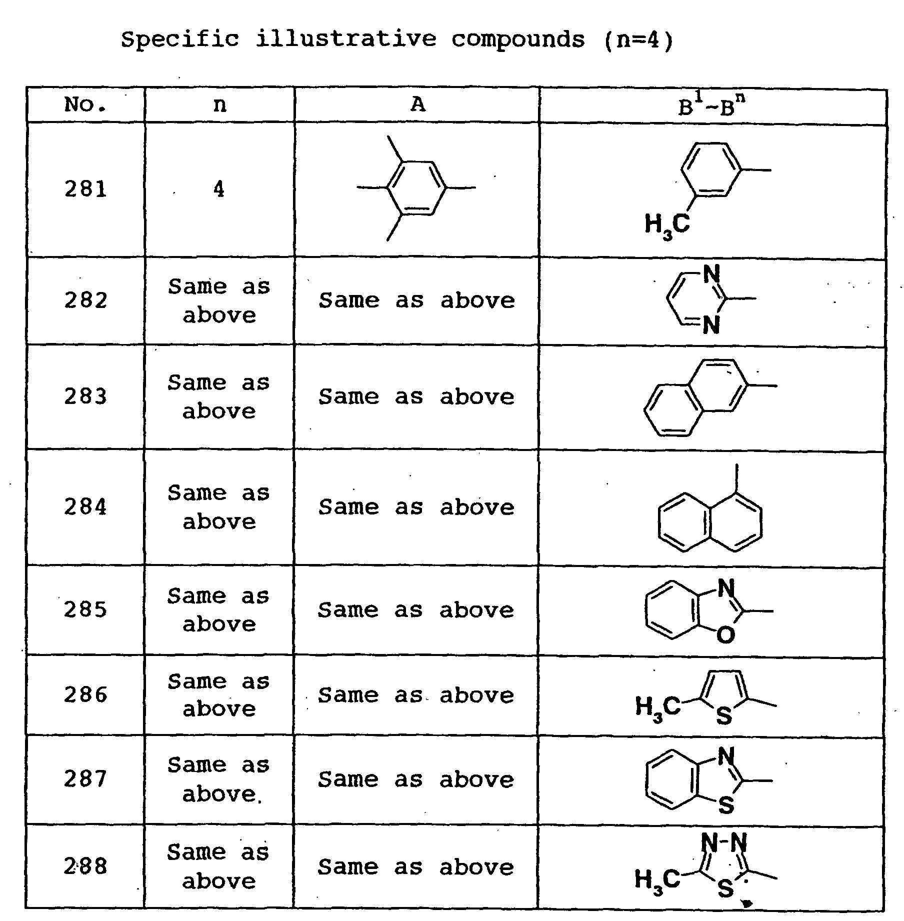

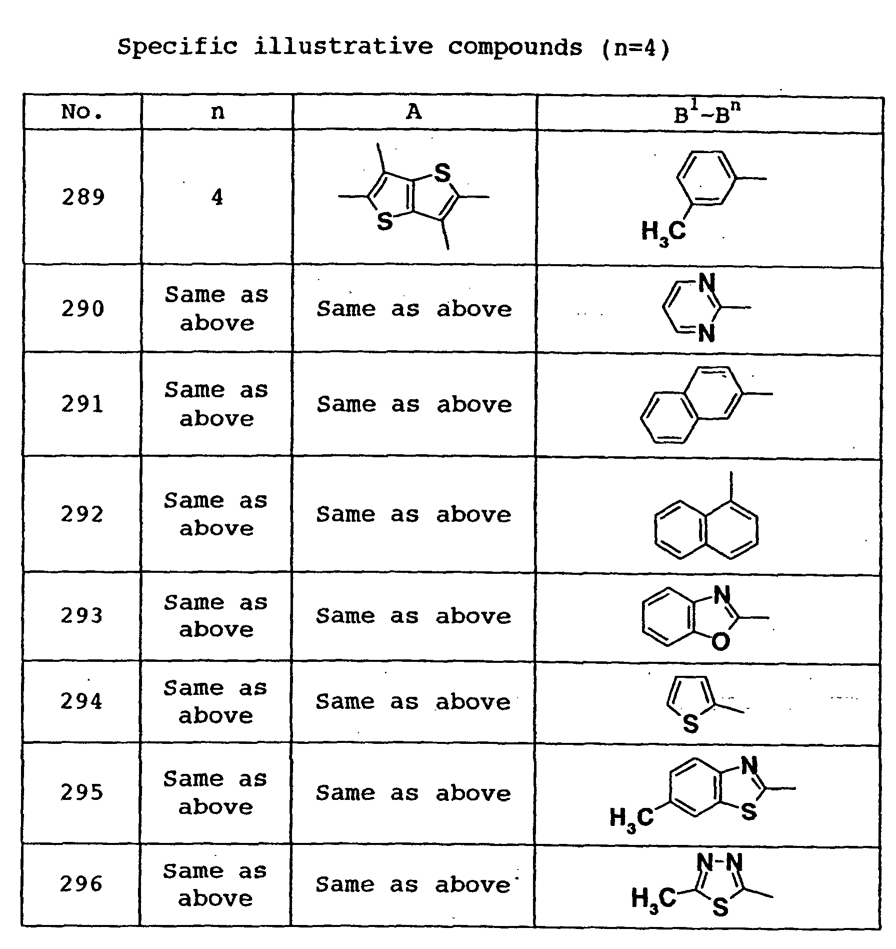

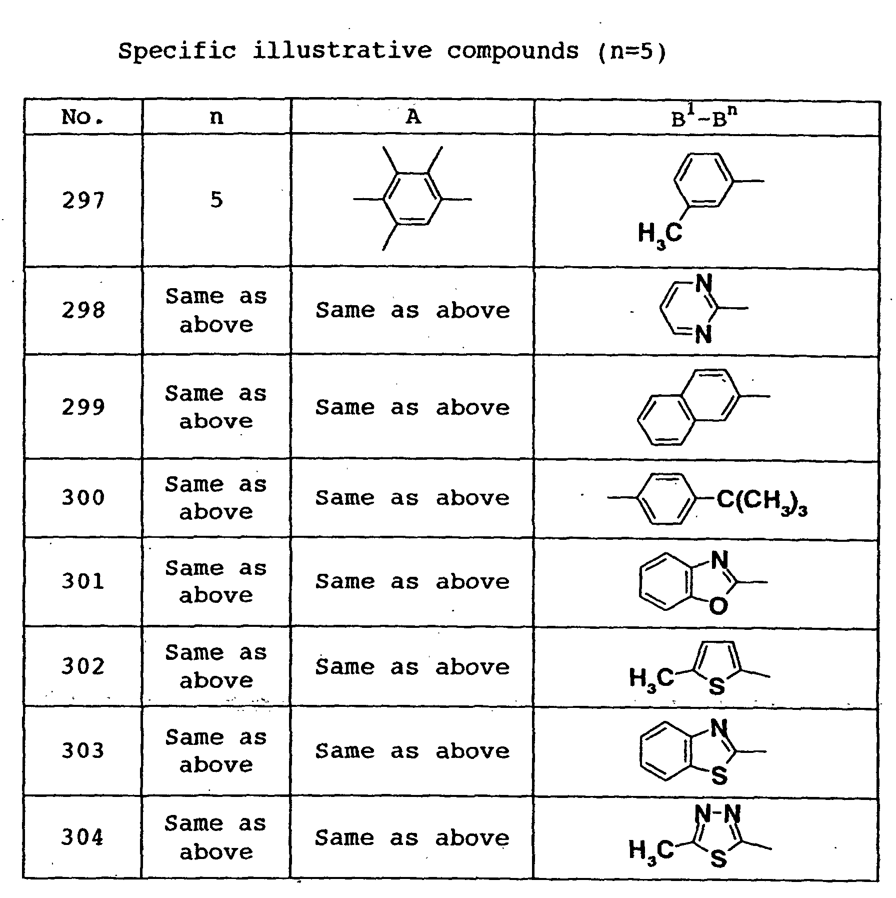

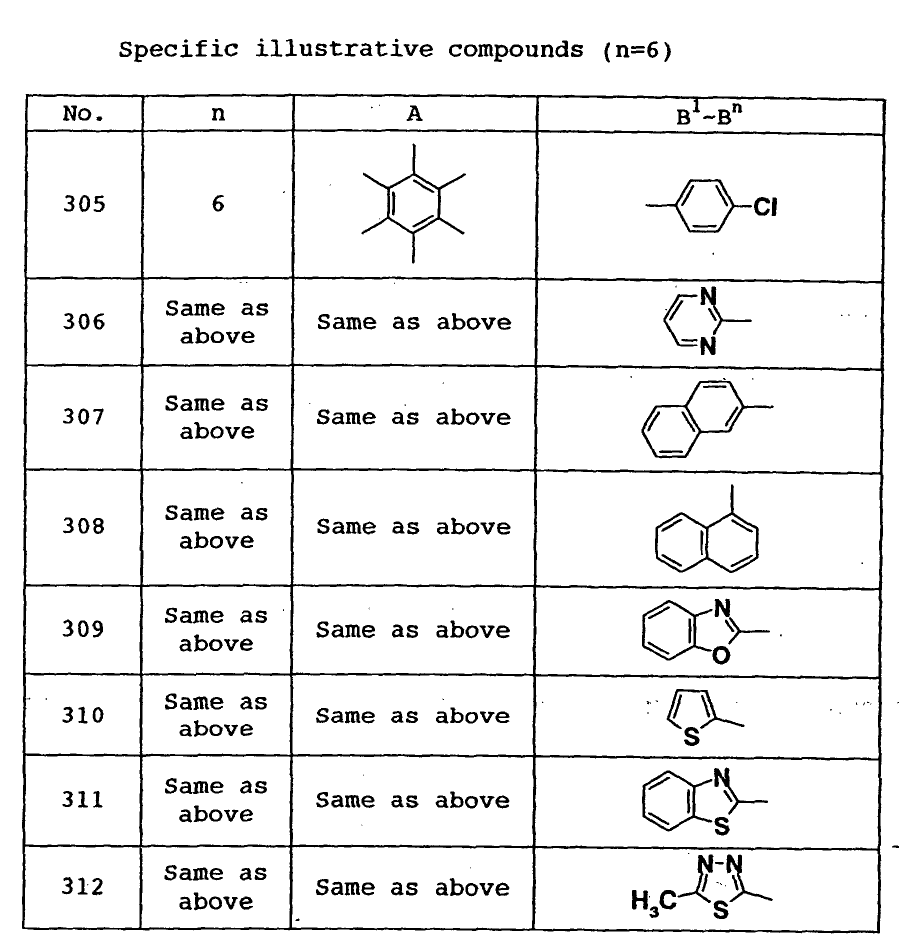

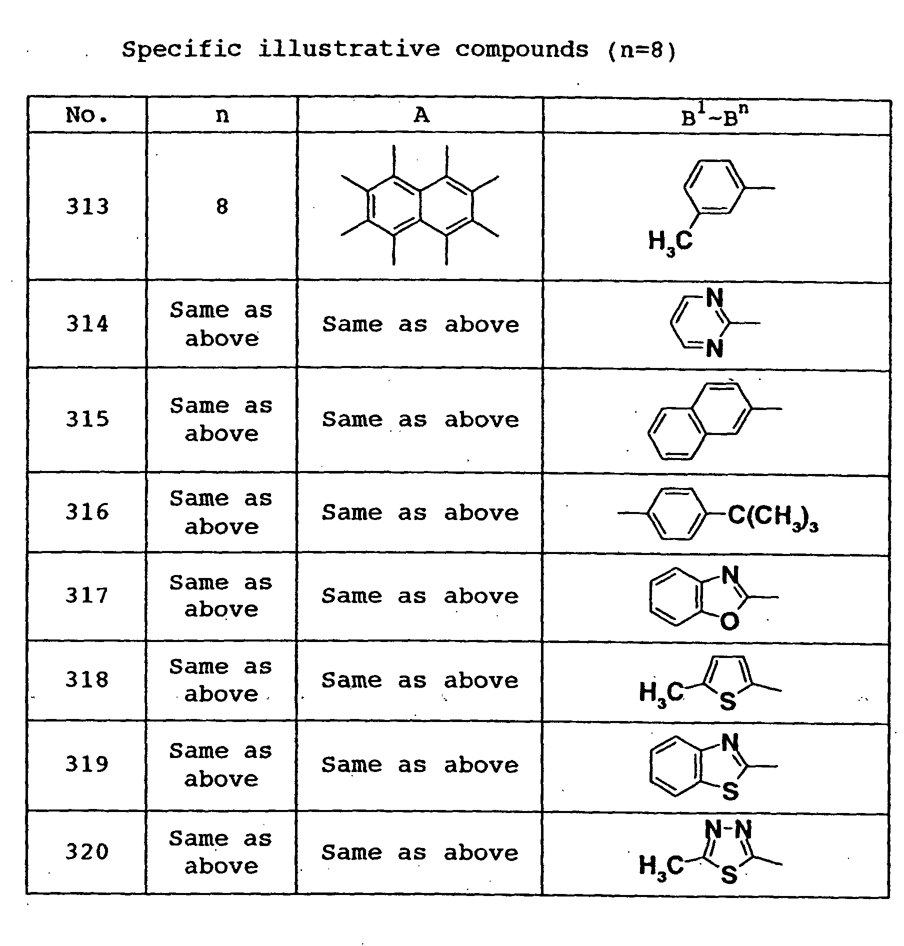

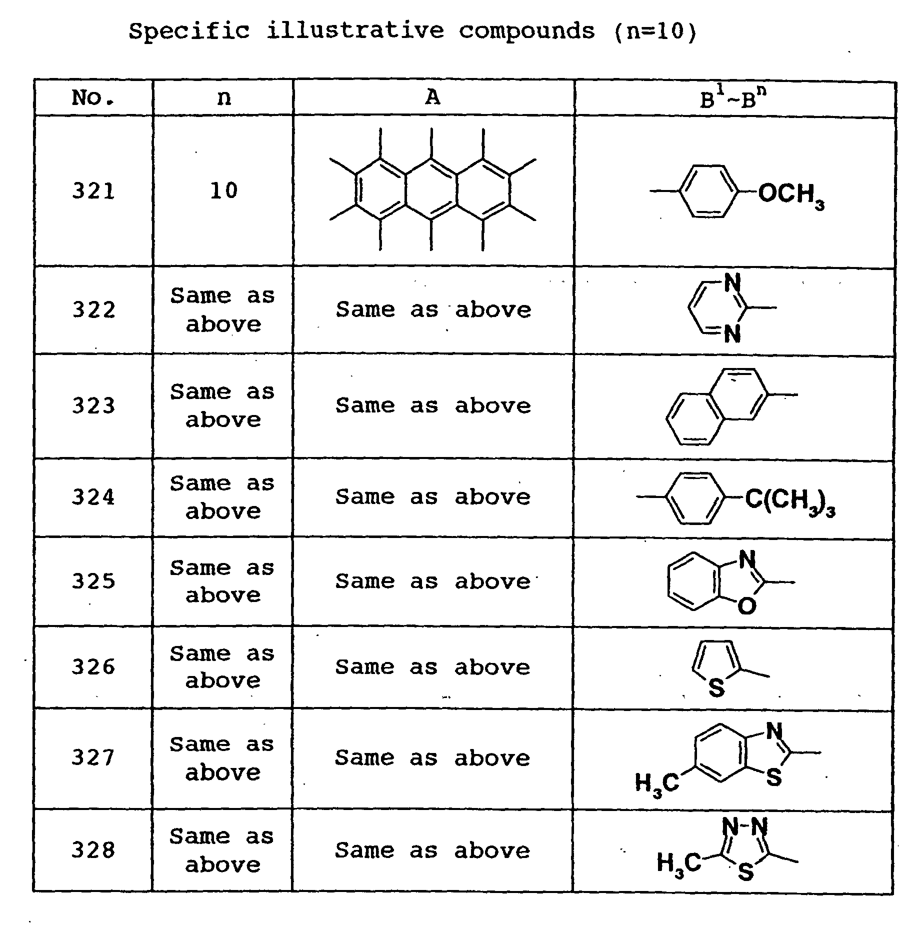

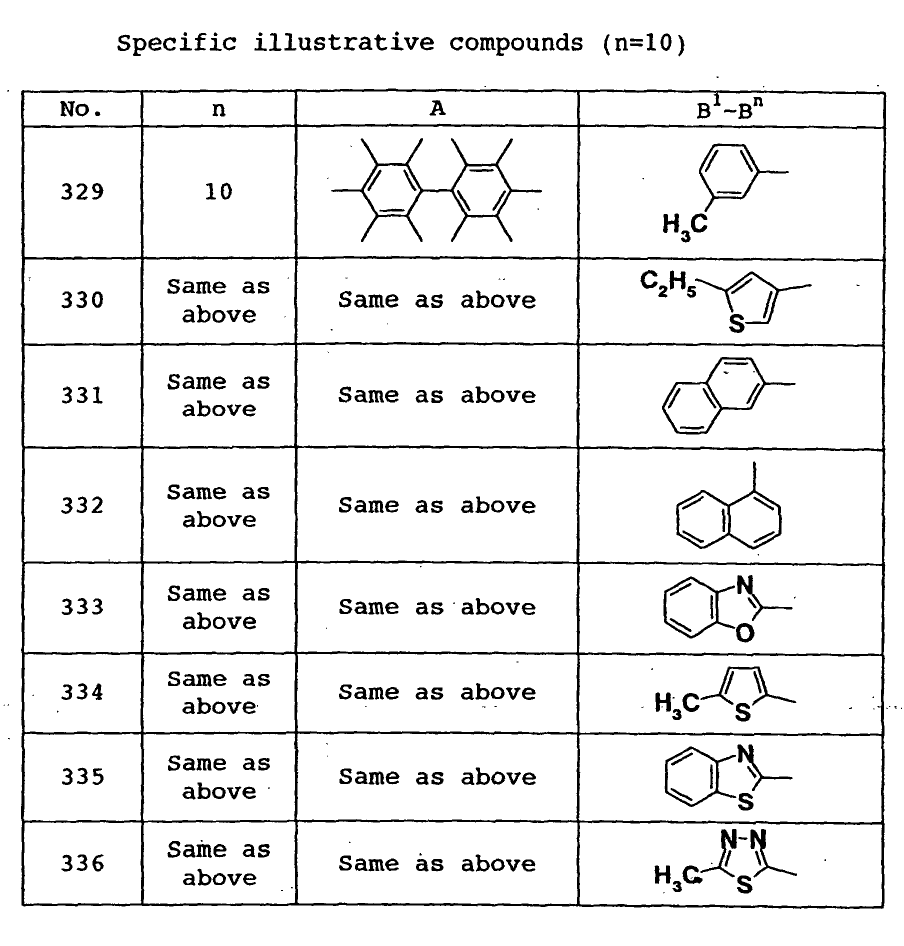

The aromatic sulfide compounds according to the present invention can each be obtained by reacting a halide and a thiol compound in the presence of a base.

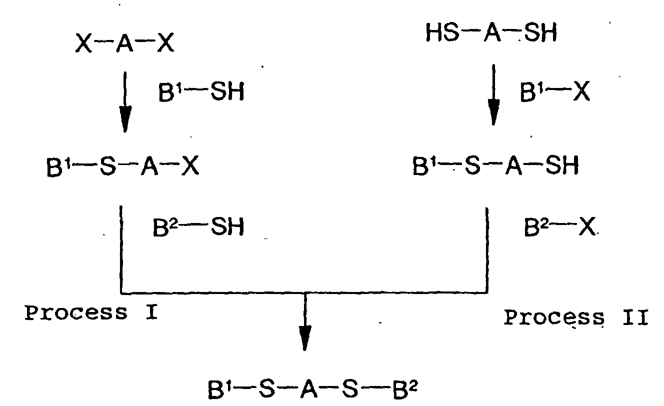

A detailed description will next be made about a production process of each aromatic sulfide compound (n=2) according to the present invention. The aromatic sulfide compound can be produced by both of the above-described synthesis routes, although its production process shall not be limited to them.

Process I will hereinafter be described in detail. Described specifically, the aromatic sulfide compound according to the present invention, which is to be included in POFs, can be obtained by reacting a dihalide and a thiol compound in the presence of a base.

The dihalide which is used in the reaction can be easily obtained by halogenating a corresponding aromatic compound.

The thiol compound which is also used in the reaction can be readily obtained by a nucleophilic displacement reaction between a diazonium salt and an anionic sulfide as disclosed, for example, in Can. J. Chem., **53**, 1480 (1975) or the like. The thiol compound can be used in a total molar proportion 2 to 5 times, preferably 2 to 3 times as much as the dihalogen compound.

Examples of the base employed in the present invention include metal hydroxides such as sodium hydroxide and potassium hydroxide, metal carbonates such as sodium carbonate and potassium carbonate, tertiary amines such as trimethylamine, triethylamine, tripropylamine, tributylamine and N,N-dimethylaniline, and metal alcoholates such as sodium methylate, sodium ethylate and potassium tert-butylate. Preferred examples are metal alcoholates such as sodium methylate and sodium ethylate.

The base can be used in a molar proportion 2 to 5 times, preferably 2 to 3 times as much as the dihalogen compound.

The reaction temperature can be in a range of from 100 to 200°C, preferably in a range of from 130 to 180°C. A reaction temperature higher than 180°C leads to an increase in byproducts, so that the yield of the target aromatic sulfide compound is lowered. A reaction temperature lower than 100°C, on the other hand, results in a slow reaction velocity and is not practical.

Use of a polar organic solvent as a reaction solvent is preferred. Illustrative of the polar organic solvent are N-methyl-2-pyrrolidone, N-propyl-2-pyrrolidone, dimethylacetamide, dimethylformamide, and dimethyl sulfoxide.

As a further production process, the aromatic sulfide compounds can also be produced by the process disclosed, for example, in Tetrahedron, Lett., **39**, 543 (1998).

It is to be noted that the above-described processes are illustrative processes for the production of aromatic sulfide compounds useful as high refractive index dopants in the present invention and that the aromatic sulfide compounds useful as high refractive index dopants in the present invention shall not be limited to those obtained only by these production processes.

The POF material according to the present invention is composed of a core part and a cladding part having a lower refractive index than a central part of the core part.

As a polymer for making up the core part of the POF according to the present invention, any polymer can be used without any particular limitation insofar as a transparent polymer can be formed. Illustrative are homopolymers or copolymers of methacrylic esters such as methyl methacrylate, ethyl methacrylate, n-propyl methacrylate, isopropyl methacrylate, n-butyl methacrylate, isobutyl methacrylate, s-butyl methacrylate, t-butyl methacrylate, cyclohexyl methacrylate, benzyl methacrylate, phenyl methacrylate, bornyl methacrylate, adamantyl methacrylate, tricyclodecyl methacrylate, dicyclopentanyl methacrylate, 2,2,2-trifluoroethyl methacrylate, 2,2,3,3-tetrafluoropropyl methacrylate, 2,2,3,3,3-pentafluoropropyl methacrylate, 2,2,3,4,4,4-hexafluorobutyl methacrylate, 1-trifluoromethyl-2,2,2-trifluoroethyl methacrylate, 1H,1H,5H-octafluoropentyl methacrylate, and blend polymers thereof; homopolymers or copolymers of aliphatically N-substituted maleimide monomers each having a methyl group, ethyl group, n-propyl group, isopropyl group, n-butyl group, isobutyl group, s-butyl group, t-butyl group, cyclohexyl group or the like as a substituent, or blend polymers thereof; and homopolymers or copolymers of styrene and derivatives thereof, and blend polymers thereof.

As a polymer for making up the cladding part of the POF according to the present invention, any polymer can be used without particular limitation insofar as a transparent polymer can be formed. Usable examples include polymethyl methacrylate (PMMA), polycarbonates (PC), and transparent copolymers between methacrylic acid or methyl methacrylate and other monomers. As such other monomers, acrylic monomers such as monofunctional (meth) acrylates, fluorinated alkyl (meth) acrylates, acrylic acid and methacrylic acid can be used.

Known processes can produce the POF according to the present invention. In general, however, it is produced by two processes, which will be exemplified hereinafter. One of these processes is to hot draw a fiber from a preform, and the other is to continuously form a fiber without going through such a preform. Incidentally, an optical material in a form before its spinning into polymer optical fibers will be defined as a "POF preform".

According to the preform process, a prefabricated hollow tube made of a polymer is filled in its hollow space with a polymerizable solution which can dissolve the polymer of the hollow tube and contains a non-polymerizable, low molecular compound in a dispersed form (i.e., a monomer mixture containing one or more monomer components, a polymerization initiator and a molecular weight modifier), the monomer(s) is polymerized from outside by applying heat or irradiating light from the outside to obtain a rod-shaped preform, and the preform is hot drawn into a desired diameter. The polymer-made hollow tube may be formed of the same monomer mixture as that filled in the hollow space except for the exclusion of the non-polymerizable, low molecular compound, or may be formed of a different monomer mixture provided that a monomer contained as a principal component is the same.

Further, as the molecular weight modifier, a conventional radical chain transfer agent, for example, a mercaptan such as n-butylmercaptan can be used. As the polymerization initiator, on the other hand, a conventional radical polymerization initiator, for example, an azo compound such as azoisobutyronitrile or peroxide such as benzoyl peroxide can be used. Here, a so-called intermediate temperature initiator capable of effectively producing radicals at about 40°C to about 100°C, such as benzoyl peroxide or lauroyl peroxide, can be suitably used. Therefore, when such an intermediate temperature initiator is used, the temperature of the polymerization reaction is suitably at about 40°C to about 100°C. To avoid development of cracks in the polymer during or-after the polymerization reaction due to heat of the reaction or an expansion or shrinkage by the reaction itself and also to prevent the monomer(s) from boiling under the heat of the reaction in the course of the reaction, it is necessary to control the velocity of the polymerization, reaction. The velocity of the polymerization reaction can be controlled by a combination of a polymerization temperature and an initiator concentration. For conditions that a radical polymerization reaction be initiated at about 40°C to about 100°C, it may be sufficient to add a radical polymerization initiator in a proportion of from 0.001 to 10 wt.% or so, more specifically from 0.01 to 0.3 wt.% or so based on the whole system. In addition to bulk polymerization by such thermal energy, bulk polymerization making use of light energy or the like is also usable. In this polymerization, the velocity of the polymerization reaction can also be controlled by a combination of a quantity of input energy such as temperature and a concentration of the initiator.

From the standpoint of the workability of drawing upon heating and melting a POF preform and spinning it into POF, the weight average molecular weight of the polymer which makes up the core part and cladding part of the POF preform may be preferably 10,000 or higher but 300,000 or lower, more preferably 30,000 or higher but 250,000 or lower, notably 50,000 or higher but 200,000 or lower.

When the core or cladding part of a plastic optical fiber material is produced by a polymerization reaction which is initiated by heating, any production system can be suitably used in the present invention irrespective of its type insofar as it can rotate a POF preform and is equipped with a heating means having a temperature-controlling function. However, the progress of this polymerization reaction may be inhibited by oxygen in air in some instances. Therefore, the production system may preferably be equipped with a function to permit sealing the POF preform at opposite ends thereof upon insertion and arrangement of the POF preform in a mold.

As the continuous process, it is possible to adopt such a procedure that a polymer of low polymerization degree, which contains a non-polymerizable compound, and a polymer of high polymerization degree, which does not contain any non-polymerizable compound, are subjected to multicomponent spinning with the latter polymer placed outside to cause diffusion of the internal non-polymerizable compound under heat.

A coating layer (jacket layer) can be arranged over an outer peripheral wall of a GI POF produced as described above. The coating layer can be formed into a multilayer structure of two or more layers. For the coating layer (jacket layer), known materials such as polyethylene, polyvinyl chloride, chlorinated polyethylene, crosslinked polyethylene, polyolefin elastomer, polyurethane, nylon resin and ethylene-vinyl acetate copolymer can be used.

The present invention will hereinafter be described specifically based on Examples.

Synthesis examples of aromatic sulfide compounds according to the present invention will be described in Examples 1-7.

Measurement of the refractive index of each optical material according to the present invention was conducted as will be described hereinafter. Compositions with a sample dispersed at varied concentrations in PMMA (product of Aldrich Chemical Co., Mw: 120,000) were spin-coated on silicon substrates, respectively, and their refractive indexes were measured by the prism coupler method (wavelength: 633 nm). From a relationship between the concentrations and the refractive indexes, the refractive index of the aromatic sulfide compound according to the present invention was calculated.

The glass transition temperature of each optical material according to the present invention was measured by DSC (manufactured by MAC Science Co., Ltd.) at a heating rate of 10°C /min.

Performance, as optical parts, of POFs making use of the aromatic sulfide compounds according to the present invention is shown in Examples 16-21. Measurement of each refractive index distribution was conducted by a known method while using "Interfaco Interference Microscope" (manufactured by Carl Zeiss Co., Ltd.). Each optical transmission loss was measured by the cutback technique while using a He-Ne laser beam (wavelength: 633 nm).

### Example 1

### Synthesis of 2,5-bis(phenylthio)thiophene

2,5-Dibrombthiophene (12.10 g, 0.050 mol), thiophenol (12.12 g, 0.110 mol) and copper(I) oxide (3.58 g, 0.025 mol) were placed in pyridine/quinoline (1/4, 100 mL), and then refluxed at 160°C for 42 hours. The reaction mixture was treated with 6N hydrochloric acid, and then extracted with toluene. The organic layer was taken out, and the solvent was eliminated by an evaporator to obtain a pale yellow liquid. The thus-obtained liquid was recrystallized from ethanol to afford the target compound. Yield: 10.1 g (67.0%). Melting point: 47-48°C.

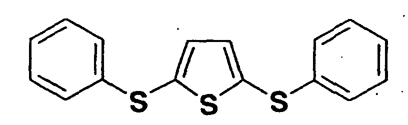

### Example 2

### Synthesis of 4,4'-bis(phenylthio)biphenyl

4,4'-Dibromobiphenyl (12.50 g, 0.040 mol), thiophenol (9.70 g, 0.088 mol) and KOH (4.94 g, 0.088 mol) were placed in DMI (100 mL), and then reacted at 160°C for 62 hours. After the reaction mixture was extracted with toluene, the solvent was eliminated to obtain a white solid. Using toluene/hexane (2/8) as a developing solvent, the white solid was purified by column chromatography to obtain a white solid. The white solid was recrystallized from IPA/ethyl acetate (9/1) to afford the target compound as a glossy, leaf-shaped, white solid. Yield: 11.5 g (78.0%). Melting point: 117.7°C.

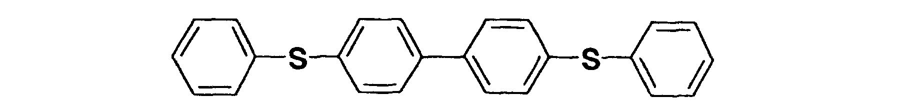

### Example 3

### Synthesis of 1,4-bis(phenylthio)benzene

p-Dibromobenzene (11.80 g, 0.050 mol), thiophenol (13.22 g, 0.120 mol) and KOH (6.73 g, 0.120 mol) were placed in DMI (100 mL), and then reacted at 160°C for 57 hours. After the reaction mixture was extracted with toluene, the solvent was eliminated to obtain a white solid. Using toluene/hexane (1/9) as a developing solvent, the white solid was purified by column chromatography to obtain a pale yellow solid. The pale yellow solid was recrystallized from ethanol to afford the target compound as a glossy, leaf-shaped, white solid. Yield: 7.18 g (49.0%). Melting point: 80-81°C.

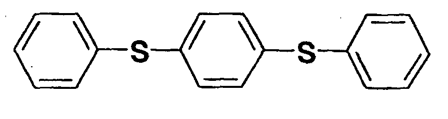

### Example 4

### Synthesis of 1,3,5-tris(phenylthio)benzene

1,3,5-Tribromobenzene (15.40 g, 0.0489 mol), thiophenol (16.43 g, 0.149 mol) and copper(I) oxide (3.56 g, 0.025 mol) were placed in pyridine/quinoline (1/4, 100 mL), and then refluxed at 160°C for 57 hours. The reaction mixture (solid) was dissolved in toluene, followed by washing with water. The resultant mixture was washed with 6N hydrochloric acid, and the toluene layer was taken out. The solvent was eliminated to obtain a pale yellow liquid. Using toluene/hexane (2/8) as a developer, column chromatography was performed to afford the target compound as a white solid. Yield: 13.0 g (66.0%). Melting point: 40-41°C.

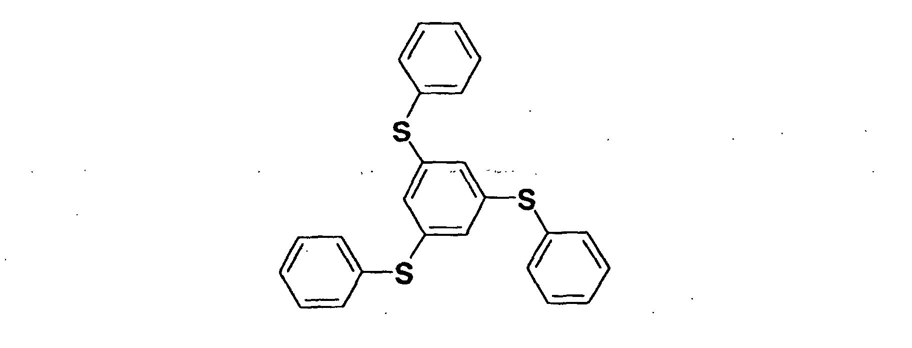

### Example 5

### Synthesis of 2,5'-bis(phenylthio)bithiophene

5,5'-Dibromo-2,2'-dithiophene (4.86 g, 0.015 mol), thiophenol (6.78 g, 0.062 mol), potassium hydroxide (4.04 g, 0.072 mol) and anhydrous DMI (50 mL) were charged into a 4-necked flask fitted with a stirrer, a thermometer and a Dimroth condenser, and then refluxed at a reaction temperature of 130°C for 13 hours and 30 minutes and further at a reaction temperature of 160°C for 6 hours and 30 minutes. Water (500 g) was added to the reaction mixture, followed by stirring. Further, toluene was added, followed by stirring. The resultant reaction mixture was allowed to separate into layers. The organic layer was washed with a saturated aqueous solution of NaCl, and then dehydrated over anhydrous magnesium sulfate. Toluene was distilled off to obtain a pale yellow solid. The solid was recrystallized and purified from IPA to afford the target compound as pale yellow needles. Yield: 5.23 g (91.1%). Melting point: 110-112°C.

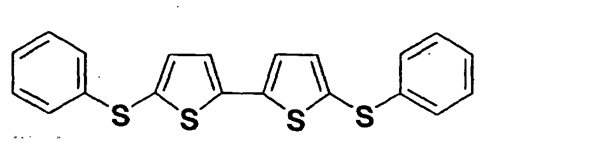

### Example 6

### Synthesis of 4,6-bis(phenylthio)pyrimidine

4,6-Dichloropyrimidine (7.45 g, 0.050 mol), thiophenol (22.12 g, 0.201 mol), potassium hydroxide (11.32 g, 0.202 mol) and anhydrous DMI (80 mL) were charged into a 4-necked flask fitted with a stirrer, a thermometer and a Dimroth condenser, and then refluxed at a reaction temperature of 130°C for 1 hour and 30 minutes and further at a reaction temperature of 150°C for 4 hours and 30 minutes. Water (1,000 g) was added to the reaction mixture, followed by stirring. Further, ethyl acetate was added, followed by stirring. The resultant reaction mixture was allowed to separate into layers. The organic layer was washed with a saturated aqueous solution of NaCl, and then dehydrated over anhydrous magnesium sulfate. Ethyl acetate was distilled off to obtain a brown mixture of a solid and a liquid. Using toluene/ethyl acetate (8/2), the liquid was purified by column chromatography to obtain a yellow solid. This solid and the above solid were recrystallized and purified from IPA to afford the target compound as pale yellow crystals. Yield: 7.75 g (52.3%). Melting point: 117°C.

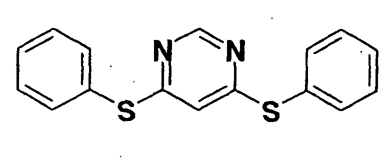

### Example 7

### Synthesis of 1,3,5-tris(phenylthio)triazine

Thiophenol (16.58 g, 0.150 mol), potassium hydroxide (9.90 g, 0.176 mol) and anhydrous DMI (80 mL) were charged into a 4-necked flask fitted with a stirrer, a thermometer and a Dimroth condenser, and then heated at a reaction temperature of 80°C for 2 hours. Cyanuric chloride (9.22 g, 0.050 mol) was added, followed by refluxing at a reaction temperature of 120°C for 3 hours and then at a reaction temperature of 140°C for 9 hours. Water was added to the reaction mixture, followed by stirring. Further, ethyl accetate was added, followed by stirring. The resultant reaction mixture was allowed to separate into layers. The organic layer was washed with a saturated aqueous solution of NaCl, and then dehydrated over anhydrous magnesium sulfate. Ethyl acetate was distilled off to obtain a yellow viscous liquid. Using toluene/hexane (6/4), the liquid was purified by column chromatography to obtain a yellow viscous liquid. (The liquid was left over for crystallization.) The thus-obtained solid was recrystallized and purified from IPA to afford the target compound as white needles. Yield: 8.79 g (43.3%). Melting point: 97-99°C.

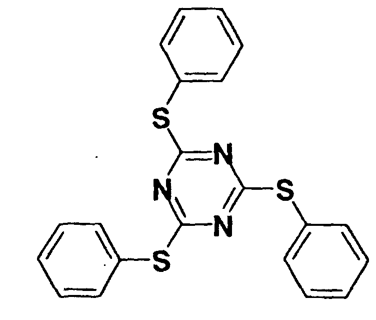

### [Measurement of Refractive Indexes]

### Example 8

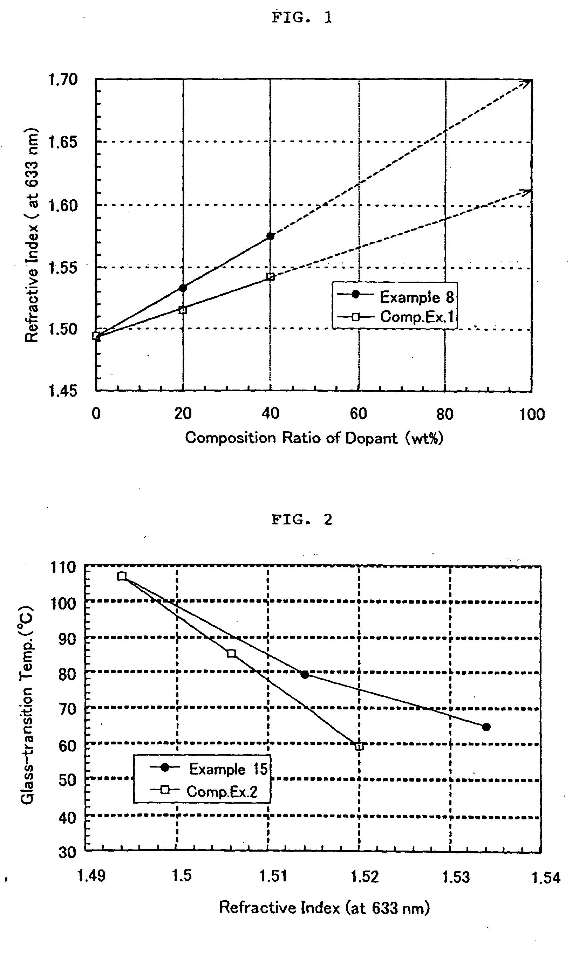

Refractive indexes of spin-coated films, which had been formed from compositions with the 1,3,5-tris(phenylthio)benzene of Example 4 dispersed at varied concentrations in PMMA, were measured by the prism coupler method. The results are plotted in FIG. 1. By extrapolation of the straight line, 1,3,5-tris(phenylthio)benzene was found to have a refractive index (n) of 1.702.

### Comparative Example 1

Refractive indexes of spin-coated films, which had been formed from compositions with diphenyl sulfide dispersed at varied concentrations in PMMA, were measured in a similar manner as in Example 8. The results are plotted in FIG. 1. By extrapolation, diphenyl sulfide was found to have a refractive index (n) of 1.615.

### Examples 9-14

In a similar manner as in Example 8, dopant concentration dependencies of refractive indexes were measured, and straight lines were extrapolated to calculate the refractive indexes of certain invention compounds. The results are shown below in Table 1. All the compounds were found to be higher in refractive index than diphenyl sulfide.

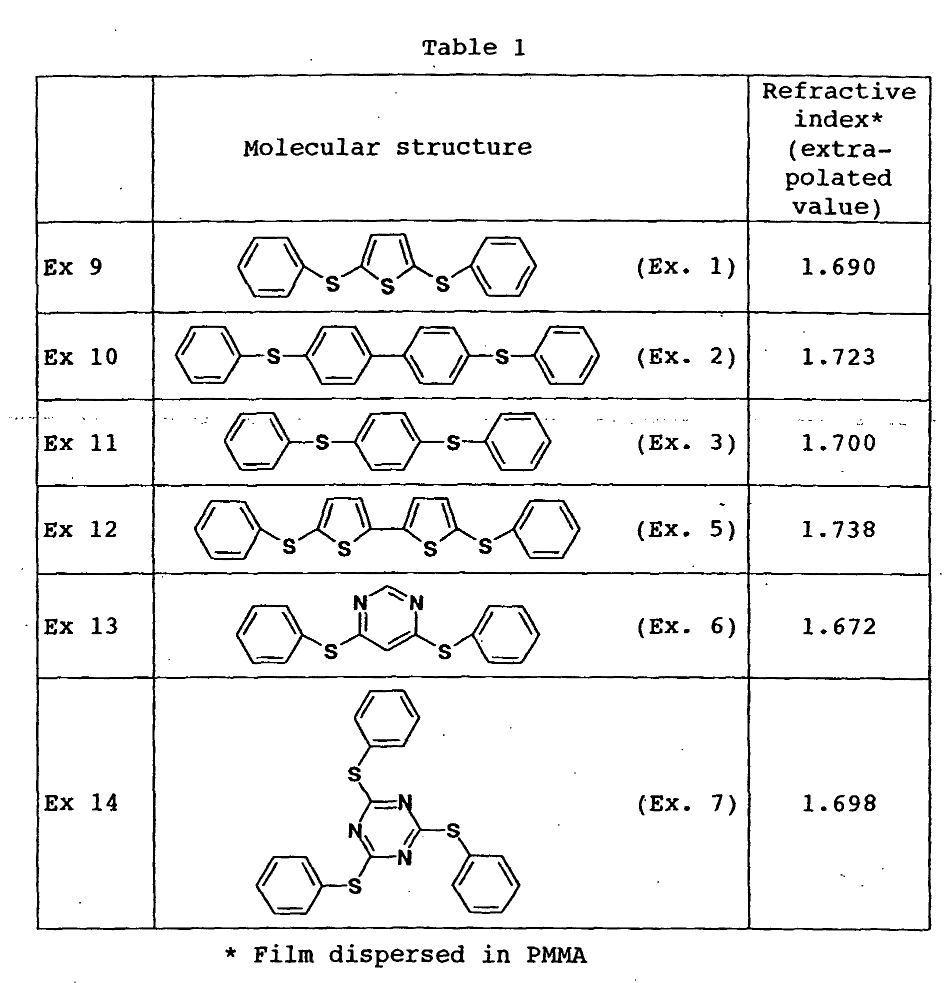

### [Measurement of Glass transition temperatures]

### Example 15

Glass transition temperatures of films, which had been formed from compositions with the 1,3,5-tris(phenylthio)benzene of Example 4 dispersed at varied concentrations in PMMA, were measured. The results of plotting of the thus-measured glass transition temperatures against the corresponding refractive indexes are shown in FIG. 2.

### Comparative Example 2

Glass transition temperatures of films, which had been formed from compositions with diphenyl sulfide dispersed at varied concentrations in PMMA, were measured in a similar manner as in Example 15. The results are plotted in FIG. 2.

### [Optical Parts]

### Example 16

A horizontally-held glass tube of 500 mm in length and 18 mm in inner diameter was filled with methyl methacrylate (MMA) (112 g), benzoyl peroxide (0.56 g) as a polymerization initiator and n-butylmercaptan (350 µL) as a chain transfer agent. After the glass tube was sealed at opposite ends thereof, the glass tube was heated at 70°C for 20 hours while rotating it at 3,000 rpm. The rotation was then stopped, and the glass tube was heated at 90°C for 10 hours to polymerize the MMA so that a polymerization tube formed of methyl methacrylate (PMMA) was prepared. A hollow part of 5 mm in diameter was centrally formed through the polymer rod to obtain a hollow tube.

The PMMA-made hollow tube was sealed at an end thereof, and then filled with MMA (48 g), the below-described dopant of high refractive index (12 g), di-t-butyl peroxide (54 µL) as a polymerization initiator and n-lauryl mercaptan (160 µL) as a chain transfer agent. After the opposite end was sealed, the tube was held horizontally. While rotating the tube at 10 rpm, the tube was heated at 95°C for 24 hours. The rotation was then stopped, and the tube was heated at 110°C for 48 hours to polymerize the MMA so that a rod of 18 mm in outer diameter was obtained.

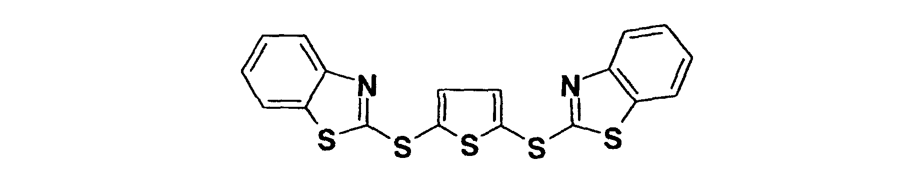

The rod was mounted upright on a rod feeder and, while heating and melting the rod in a cylindrical heating furnace controlled at 220°C, was drawn and taken up at a constant speed so that the rod was melt spun to obtain an optical fiber of 0.75 mm in diameter. The refractive index distribution of a section of the thus-obtained optical fiber was measured. The refractive index was found to continuously decrease from a central part toward an outer periphery. Transmission characteristics of the thus-obtained optical fiber over a length of 100 m were evaluated. Its transmission loss was 17.8 dB at a wavelength of 650 nm, while its transmission band was 3.4 GHz. The optical fiber, therefore, had good performance as POF with distributed refractive index. Further, the thus-obtained optical fiber was placed in an oven controlled at 85°C and a heating test was conducted. The refractive index distribution after 3,000 hours was measured. The optical fiber was found to still retain the initial refractive index distribution.

### Example 17

A PMMA-made hollow tube prepared in a similar manner as in Example 16 was provided. The PMMA-made hollow tube was filled with MMA (48 g), the below-described dopant of high refractive index (12 g), di-t-butyl peroxide (54 µL) as a polymerization initiator and n-lauryl mercaptan (160 µL) as a chain transfer agent. After the opposite end was sealed, the tube was held horizontally. While rotating the tube at 10 rpm, the tube was heated at 95°C for 24 hours. The rotation was then stopped, and the tube was heated at 110°C for 48 hours to polymerize the MMA so that a rod of 18 mm in outer diameter was obtained.

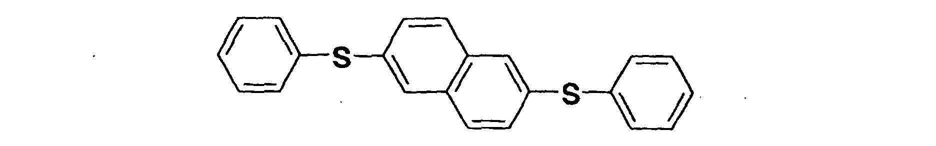

The rod was mounted upright on a rod feeder and, while heating and melting the rod in a cylindrical heating furnace controlled at 220°C, was drawn and taken up at a constant speed so that the rod was melt spun to obtain an optical fiber of 0.75 mm in diameter. The refractive index distribution of a section of the thus-obtained optical fiber was measured. The refractive index was found to gradually decrease from a central part toward an outer periphery. Transmission characteristics of the thus-obtained optical fiber over a length of 100 m were evaluated. Its transmission loss was 15.3 dB at a wavelength of 650 nm, while its transmission band was 3.1 GHz. The optical fiber, therefore, had good performance as a plastic optical fiber with distributed refractive index. Further, the thus-obtained optical fiber was placed in an oven controlled at 85°C and a heating test was conducted. The refractive index distribution after 3,000 hours was measured. The optical fiber was found to still retain the initial refractive index distribution.

### Example 18

A PMMA-made hollow tube prepared in a similar manner as in Example 16 was provided. The PMMA-made hollow tube was filled with MMA (48 g), the below-described dopant of high refractive index (12 g), di-t-butyl peroxide (54 µL) as a polymerization initiator and n-lauryl mercaptan (160 µL) as a chain transfer agent. After the opposite end was sealed, the tube was held horizontally. While rotating the tube at 10 rpm, the tube was heated at 95°C for 24 hours. The rotation was then stopped, and the tube was heated at 110°C for 48 hours to polymerize the MMA so that a rod of 17.6 mm in outer diameter was obtained.

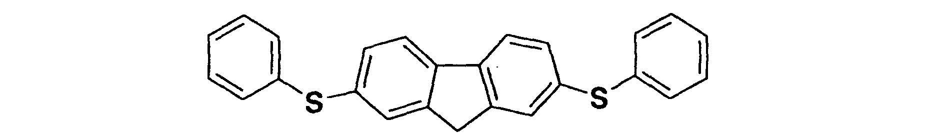

The rod was mounted upright on a rod feeder and, while heating and melting the rod in a cylindrical heating furnace controlled at 220°C, was drawn and taken up at a constant speed so that the rod was melt spun to obtain an optical fiber of 0.75 mm in diameter. The refractive index distribution of a section of the thus-obtained optical fiber was measured. The refractive index was found to gradually decrease from a central part toward an outer periphery. Transmission characteristics of the thus-obtained optical fiber over a length of 100 m were evaluated. Its transmission loss was 14.5 dB at a wavelength of 650 nm, while its transmission band was 2.3 GHz. The optical fiber, therefore, had good performance as a plastic optical fiber with distributed refractive index. Further, the thus-obtained optical fiber was placed in an oven controlled at 85°C and a heating test was conducted. The refractive index distribution after 3,000 hours was measured. The optical fiber was found to still retain the initial refractive index distribution.

### Example 19

A PMMA-made hollow tube prepared in a similar manner as in Example 16 was provided. The PMMA-made hollow tube was filled with MMA (48 g), the below-described dopant of high refractive index (12 g), di-t-butyl peroxide (54 µL) as a polymerization initiator and n-lauryl mercaptan (160 µL) as a chain transfer agent. After the opposite end was sealed, the tube was held horizontally. While rotating the tube at 10 rpm, the tube was heated at 95°C for 24 hours. The rotation was then stopped, and the tube was heated at 110°C for 48 hours to polymerize the MMA so that a rod of 18 mm in outer diameter was obtained.

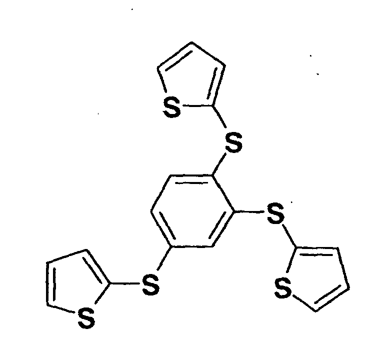

The rod was mounted upright on a rod feeder and, while heating and melting the rod in a cylindrical heating furnace controlled at 220°C, was drawn and taken up at a constant speed so that the rod was melt spun to obtain an optical fiber of 0.75 mm in diameter. The refractive index distribution of a section of the thus-obtained optical fiber was measured. The refractive index was found to continuously decrease from a central part toward an outer periphery. Transmission characteristics of the thus-obtained optical fiber over a length of 100 m were evaluated. Its transmission loss was 17.8 dB at a wavelength of 650 nm, while its transmission band was 3.5 GHz. The optical fiber, therefore, had good performance as POP with distributed refractive index. Further, the thus-obtained optical fiber was placed in an oven controlled at 85°C and a heating test was conducted. The refractive index distribution after 3,000 hours was measured. The optical fiber was found to still retain the initial refractive index distribution.

### Example 20

A PMMA-made hollow tube prepared in a similar manner as in Example 16 was provided. The PMMA-made hollow tube was filled with MMA (48 g), the below-described dopant of high refractive index (12 g), di-t-butyl peroxide (54 µL) as a polymerization initiator and n-lauryl mercaptan (160 µL) as a chain transfer agent. After the opposite end was sealed, the tube was held horizontally. While rotating the tube at 10 rpm, the tube was heated at 95°C for 24 hours. The rotation was then stopped, and the tube was heated at 110°C for 48 hours to polymerize the MMA so that a rod of 18 mm in outer diameter was obtained.

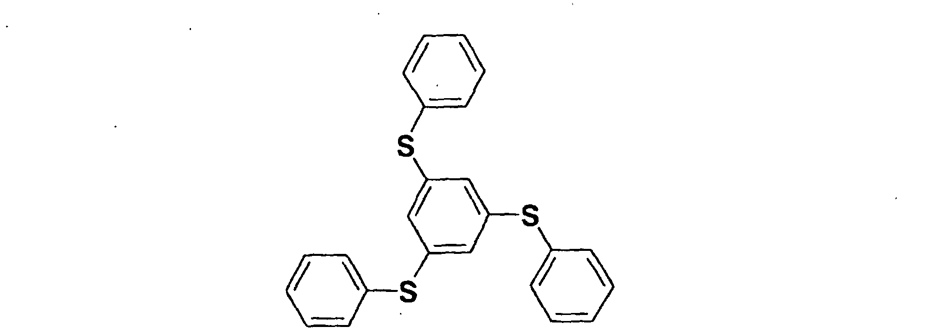

The rod was mounted upright on a rod feeder and, while heating and melting the rod in a cylindrical heating furnace controlled at 220°C, was drawn and taken up at a constant speed so that the rod was melt spun to obtain an optical fiber of 0.75 mm in diameter. The refractive index distribution of a section of the thus-obtained optical fiber was measured. The refractive index was found to gradually decrease from a central part toward an outer periphery. Transmission characteristics of the thus-obtained optical fiber over a length of 100 m were evaluated. Its transmission loss was 16.2 dB at a wavelength of 650 nm, while its transmission band was 3.1 GHz. The optical fiber, therefore, had good performance as a plastic optical fiber with distributed refractive index. Further, the thus-obtained optical fiber was placed in an oven controlled at 85°C and a heating test was conducted. The refractive index distribution after 3,000 hours was measured. The optical fiber was found to still retain the initial refractive index distribution.

### Example 21

The 2,5-bis(phenylthio)thiophene of Example 1 was added at 20 wt.% to PMMA, and they were mixed for 10 minutes in a mortar. The sample was formed into a film by a hot press, and its optical properties were measured. The film so obtained was found to have a whole light transmittance of 91%, a hue of 3.5, nd of 1.5187, and an Abbe number of 46.7. 2.5-Bis(phenylthio)thiophene was, therefore, found to increase the refractive index of PMMA without substantially changing the transmittance and hue of PMMA alone.

### INDUSTRIAL APPLICABILITY

The optical materials according to the present invention can bring about high refractive indexes more efficiently than the dopants known to date. They have smaller plasticizing effect and are excellent in heat' resistance, so that they are equipped with improved reliability as optical materials.

Further, GI POF, one of optical parts according to the present invention, is excellent in refractive index distribution and heat resistant stability compared with conventional GI POFs, and is equipped with transmission characteristics of improved reliability as a optical' fiber. Accordingly, POFs according to the present invention can also be used over an extended period of time in fields where heat resistance is required, such as automobile engine compartments and the like.

## Claims

1. An optical material comprising at least one aromatic sulfide compound represented by the following formula (1): 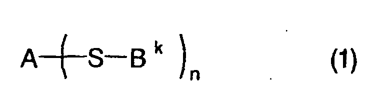 wherein
n stands for an integer of from 2 to 12,
k stands for an integer of from 1 to n,
A represents a substituted or unsubstituted, n-valent carbocyclic aromatic ring or heterocyclic aromatic ring, and
B¹ to Bⁿ each independently represent a substituted or unsubstituted, carbocyclic aromatic group or heterocyclic aromatic group.

2. An optical material according to claim 1, wherein in formula (1), n stands for an integer of from 2 to 4, and A is a substituted or unsubstituted, heterocyclic aromatic ring.

3. An optical material according to claim 2, wherein in formula (1), B¹ to Bⁿ each independently area substituted or unsubstituted phenyl group, a substituted or unsubstituted pyrimidyl group, a substituted or unsubstituted naphthyl group, a substituted or unsubstituted thienyl group, a substituted or unsubstituted benzothiazolyl group, a substituted or unsubstituted benzoxazolyl group, a substituted or unsubstituted thiadiazolyl group, or a substituted or unsubstituted thiazolyl group.

4. An optical material according to claim 2, wherein in formula (1), A is a divalent heterocyclic aromatic ring selected from a substituted or unsubstituted thiophene ring, a substituted or unsubstituted thiophene-1,1-dioxide ring, a substituted or unsubstituted thiophenethiadiazole ring, a substituted or unsubstituted thieno[3,2,-b]thiophene ring, a substituted or unsubstituted triazine ring, or a substituted or unsubstituted pyrimidine ring.

5. An optical material according to claim 4, wherein in formula (1), B¹ to Bⁿ each independently are a substituted or unsubstituted phenyl group, a substituted or unsubstituted pyrimidyl group, a substituted or unsubstituted naphthyl group, a substituted or unsubstituted thienyl group, a substituted or unsubstituted benzothiazolyl group, a substituted or unsubstituted benzoxazolyl group, a substituted or unsubstituted thiadiazolyl group, or a substituted or unsubstituted thiazolyl group.

6. An optical material according to claim 2, wherein in formula (1), A is a trivalent heterocyclic aromatic ring selected from a substituted or unsubstituted thiophene ring, a substituted or unsubstituted triazine ring, or a substituted or unsubstituted pyrimidine ring.

7. An optical material according to claim 6, wherein in formula (1), B¹ to Bⁿ each independently are a substituted or unsubstituted phenyl group, a substituted or unsubstituted pyrimidyl group, a substituted or unsubstituted naphthyl group, a substituted or unsubstituted thienyl group, a substituted or unsubstituted benzothiazolyl group, a substituted or unsubstituted benzoxazolyl group, a substituted or unsubstituted thiadiazolyl group, or a substituted or unsubstituted thiazolyl group.

8. An optical material according to claim 2, wherein in formula (1), A is a tetravalent heterocyclic aromatic ring selected from a substituted or unsubstituted thiophene ring or a substituted or unsubstituted thieno[3,2,-b]thiophene ring.

9. An optical material according to claim 8, wherein in formula (1), B¹ to Bⁿ each independently are a substituted or unsubstituted phenyl group, a substituted or unsubstituted pyrimidyl group, a substituted or unsubstituted naphthyl group, a substituted or unsubstituted thienyl group, a substituted or unsubstituted benzothiazolyl group, a substituted or unsubstituted benzoxazolyl group, a substituted or unsubstituted thiadiazolyl group, or a substituted or unsubstituted thiazolyl group.

10. An optical material according to claim 1, wherein in formula (1), n stands for an integer of from 2 to 6, and A is a substituted or unsubstituted, carbocyclic aromatic ring.

11. An optical material according to claim 10, wherein in formula (1), B¹ to Bⁿ each independently are a substituted or unsubstituted phenyl group, a substituted or unsubstituted pyrimidyl group, a substituted or unsubstituted naphthyl group, a substituted or unsubstituted thienyl group, a substituted or unsubstituted benzothiazolyl group, a substituted or unsubstituted benzoxazolyl group, a substituted or unsubstituted thiadiazolyl group, or a substituted or unsubstituted thiazolyl group.

12. An optical material according to claim 10, wherein in formula (1), A is a divalent carbocyclic aromatic ring selected from a substituted or unsubstituted benzene ring, a substituted or unsubstituted naphthalene ring, a substituted or unsubstituted fluorene ring, or a substituted or unsubstituted biphenyl group.

13. An optical material according to claim 12, wherein in formula (1), B¹ to Bⁿ each independently are a substituted or unsubstituted phenyl group, a substituted or unsubstituted pyrimidyl group, a substituted or unsubstituted naphthyl group, a substituted or unsubstituted thienyl group, a substituted or unsubstituted benzothiazolyl group, a substituted or unsubstituted benzoxazolyl group, a substituted or unsubstituted thiadiazolyl group, or a substituted or unsubstituted thiazolyl group.

14. An optical material according to claim 10, wherein in formula (1), A is a trivalent carbocyclic aromatic ring selected from a substituted or unsubstituted benzene ring or a substituted or unsubstituted fluorene ring.

15. An optical material according to claim 14, wherein in formula (1), B¹ to Bⁿ each independently are a substituted or unsubstituted phenyl group, a substituted or unsubstituted pyrimidyl group, a substituted or unsubstituted naphthyl group, a substituted or unsubstituted thienyl group, a substituted or unsubstituted benzothiazolyl group, a substituted or unsubstituted benzoxazolyl group, a substituted or unsubstituted thiadiazolyl group, or a substituted or unsubstituted thiazolyl group.

16. An optical material according to claim 10, wherein in formula (1), A is a tetravalent carbocyclic aromatic ring selected from a substituted or unsubstituted benzene ring or a substituted or unsubstituted biphenyl group.

17. An optical material according to claim 16, wherein in formula (1), B¹ to Bⁿ each independently is a substituted or unsubstituted phenyl group, a substituted or unsubstituted pyrimidyl group, a substituted or unsubstituted naphthyl group, a substituted or unsubstituted thienyl group, a substituted or unsubstituted benzothiazolyl group, a substituted or unsubstituted benzoxazolyl group, a substituted or unsubstituted thiadiazolyl group, or a substituted or unsubstituted thiazolyl group.

18. An optical material according to claims 1 to 17, which is a polymer optical fiber material.

19. An optical part comprising a polymer optical fiber material according to claim 18.

20. An optical part according to claim 19, which is a GI polymer optical fiber.

21. An aromatic sulfide compound represented by the following formula (1a): 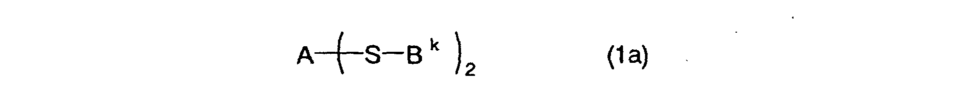 wherein
k stands for an integer of from 1 to 2,
A represents a divalent carbocyclic aromatic ring or heterocyclic aromatic ring selected from a substituted or unsubstituted benzene ring, a substituted or unsubstituted naphthalene ring, a substituted or unsubstituted fluorene ring, a substituted or unsubstituted biphenyl ring, a substituted or unsubstituted thiophene ring, a substituted or unsubstituted thiophene-1,1-dioxide ring, a substituted or unsubstituted thiophenethiadiazole ring, a substituted or unsubstituted thieno[3,2,-b]thiophene ring, a substituted or unsubstituted triazine ring, or a substituted or unsubstituted pyrimidine ring, and
B¹ to Bⁿ each independently represent a carbocyclic aromatic group or heterocyclic aromatic group selected from a substituted or unsubstituted phenyl group, a substituted or unsubstituted pyrimidyl group, a substituted or unsubstituted naphthyl group, a substituted or unsubstituted thienyl group, a substituted or unsubstituted benzothiazolyl group, a substituted or unsubstituted benzoxazolyl group, a substituted or unsubstituted thiadiazolyl group, or a substituted or unsubstituted thiazolyl group.

22. An aromatic sulfide compound represented by the following formula (1b): 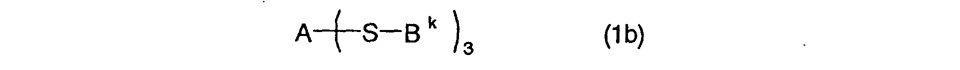 wherein
k stands for an integer of from 1 to 3,
A represents a trivalent carbocyclic aromatic ring or heterocyclic aromatic ring selected from a substituted or unsubstituted benzene ring, a substituted or unsubstituted fluorene ring, a substituted or unsubstituted thiophene ring, a substituted or unsubstituted triazine ring, or a substituted or unsubstituted pyrimidine ring, and
B¹, B² and B³ each independently represent a carbocyclic aromatic group or heterocyclic aromatic group selected from a substituted or unsubstituted phenyl group, a substituted or unsubstituted pyrimidyl group, a substituted or unsubstituted naphthyl group, a substituted or unsubstituted thienyl group, a substituted or unsubstituted benzothiazolyl group, a substituted or unsubstituted benzoxazolyl group, a substituted or unsubstituted thiadiazolyl group, or a substituted or unsubstituted thiazolyl group.

23. An aromatic sulfide compound represented by the following formula (1c): 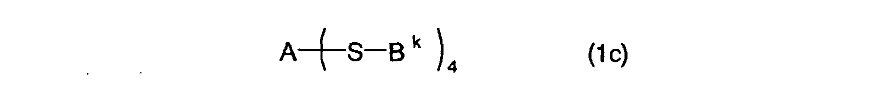 wherein
k stands for an integer of from 1 to 4,
A represents a carbocyclic aromatic ring or heterocyclic aromatic ring selected from a substituted or unsubstituted benzene ring, a substituted or unsubstituted biphenyl ring, a substituted or unsubstituted thiophene ring, a substituted or unsubstituted thieno[3,2,-b]thiophene ring, and
B¹, B², B³ and B⁴ each independently represent a carbocyclic aromatic group or heterocyclic aromatic group selected from a substituted or unsubstituted phenyl group, a substituted or unsubstituted pyrimidyl group, a substituted or unsubstituted naphthyl group, a substituted or unsubstituted thienyl group, a substituted or unsubstituted benzothiazolyl group, a substituted or unsubstituted benzoxazolyl group, a substituted or unsubstituted thiadiazolyl group, or a substituted or unsubstituted thiazolyl group.
